(19) Europäisches Patentamt
European Patent Office
Office européen des brevets

(11) Publication number: **0 359 400 A1**

# (12) EUROPEAN PATENT APPLICATION

(21) Application number: 89308246.1

(22) Date of filing: 15.08.89

(51) Int. Cl.5: C07D 319/20, A01N 43/32, C07D 327/06, C07D 405/06, C07D 413/06

(30) Priority: 16.08.88 US 232682

(43) Date of publication of application: 21.03.90 Bulletin 90/12

(84) Designated Contracting States: ES GR

(71) Applicant: E.I. DU PONT DE NEMOURS AND COMPANY
1007 Market Street
Wilmington Delaware 19898(US)

(72) Inventor: Selby, Thomas Paul
116 Hunter Court
Wilmington Delaware 19808(US)

(74) Representative: Hildyard, Edward Martin et al
Frank B. Dehn & Co. European Patent Attorneys Imperial House 15-19 Kingsway
London WC2B 6UZ(GB)

## (54) Fungicidal benzodioxane amine derivatives.

(57) Fungal diseases in plants are controlled by applying to the locus of the plant a compound of the formula:

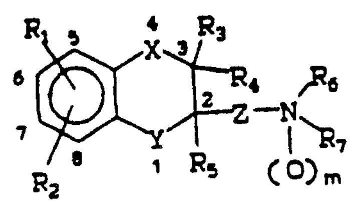

I

wherein
$R_1$ and $R_2$ are independently H, halogen or an organic group;
$R_3$, $R_4$ and $R_5$ are independently H, $CH_3$, $CF_3$ or $C_2H_5$;
$R_6$ and $R_7$ are alkyl or together form a heterocyclic ring, e.g. piperidino;
X and Y are independently O or S;
Z is unbranched alkylene;
or an agriculturally suitable salt thereof.

EP 0 359 400 A1

# FUNGICIDAL BENZODIOXANE AMINE DERIVATIVES

## BACKGROUND OF THE INVENTION

### Field of the Invention

This invention relates to novel fungicidal benzodioxane amine derivatives used for controlling fungal diseases, particularly powdery mildews, in plants.

### Description of Related Art

GB-A-2,177,084, published January 14, 1987, discloses fungicidal compositions of compounds of the formula

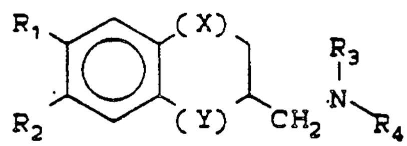

wherein
X is $CH_2$ and Y is O; or
X is O and Y is $CH_2$ or CHOH.

GB-A-2,176,780, published January 7, 1987, discloses fungicidal compositions of compounds of the formula

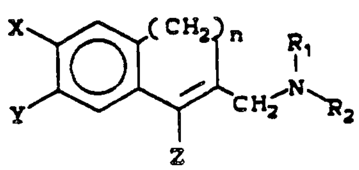

I

wherein
Z is H or $C_1$-$C_4$ alkyl; and
n is O or 1.

GB-A-1,176,782, published January 7, 1987, discloses fungicidal compositions of compounds of the formula

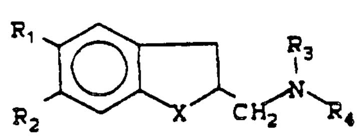

wherein X is O or S.

GB-A-2,177,085, published January 14, 1987, discloses fungicidal compositions of compounds of the formula

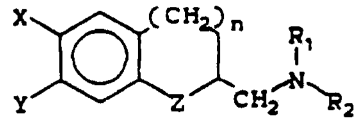

wherein
Z is C(O), $CR_3R_4$ or $CR_3OR_4$; and
n is O or 1.

U.S. 4,129,655, issued December 12, 1978 and U.S. 4,104,396, issued August 1, 1978, disclosure neuroleptic 1-piperidinoalkyl-1,4-benzodioxans of the formula

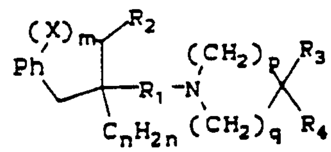

wherein
Ph is optionally substituted 1,2-phenylene.

U.S. 2,366,611, issued January 2, 1945, discloses benzodioxane derivatives of the formula

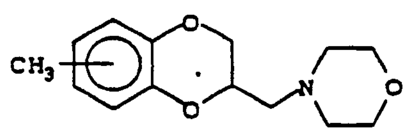

as therapeutic agents and therapeutical intermediates.

U.S. 2,056,046, issued September 29, 1936, discloses a process for the preparation of benzodioxanes of the formula

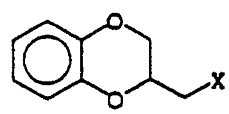

wherein
X is NHR, $NRR_1$,

or 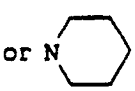

having paralyzing action on the nervous system.

U.S. 4,039,676, issued August 2, 1977, discloses (piperidinoalkyl)benzodioxans of the formula

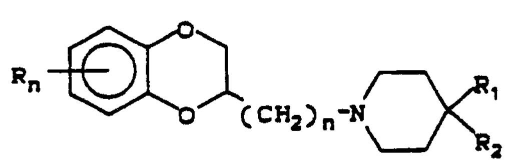

as analgesics and tranquilizers.

Croat. Chem. Acta 29,363-7 (1957) (CA 53:16137c) discloses 7-substituted -2-aminomethyl-1,4-benzodioxan derivatives with sedative action.

Zh. Organ. Khim. 2(2),307-10 (1966) (CA 65:2252e) discloses 3,3-dimethyl-2-(1-aminoethyl)benzo-1,4-dioxanes of the formula

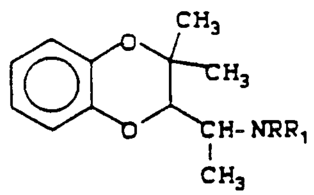

J. Org. Chem. 26,339-43 (1961) (CA 55:13431a) discloses 3-methyl-1,4-benzodioxan-2-amines and their salts.

Therapie 17(4), 599-608 (1962) (CA 68:16673g) discloses alkylamino-methylbenzodioxan derivatives and their adrenergic and cholinergic effects.

Rend. ist. super. sanita 22, 207-16 (1959) (CA 54:1522e) discloses 2-aminoethyl-1,4-benzodioxan derivatives.

Rend. ist. super. sanita 22, 217-24 (1959) (CA 54:1523e) discloses 2-aminopropyl-1,4-benzodioxan derivatives.

U.S. 3,513,239, issued May 12, 1970, discloses antidepressant 2-aminoalkyl compounds of the formula

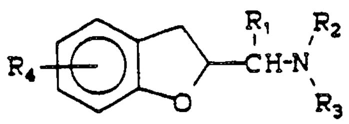

wherein

$R_1$ is H or lower alkyl;

$R_2$ and $R_3$ are each H or lower alkyl; or

$R_2$ and $R_3$ may be taken together as a 5- to 6-membered basic heterocyclic ring;

$R_4$ is H, $C_1$-$C_3$ alkyl, $C_1$-$C_3$ alkoxy or halogen.

J. Med. Pharm. Chem. 5, 1285-93 (1962) (CA 38:2805g) discloses pharmacologically active 1,4-benzodioxans of the formula

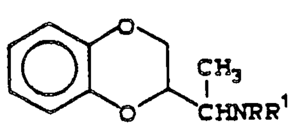

## SUMMARY OF THE INVENTION

This invention comprises a method for controlling fungal diseases in plants comprising applying to the locus of the plant a compound of formula I

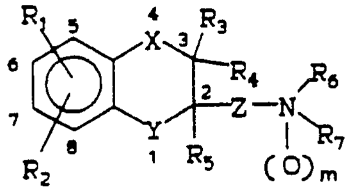

I

wherein

$R_1$ and $R_2$ are independently H, halogen, $C_1$-$C_8$ alkyl, $C_2$-$C_8$ alkenyl, $C_2$-$C_8$ alkynyl, $C_1$-$C_8$ alkoxy, $C_1$-$C_5$ alkyl substituted with 1-3 F, Cl or Br or $C_2$-$C_4$ alkoxyalkyl, $Si(CH_3)_3$, thioalkyl, or phenyl substituted with one or two of the following: halogen, $C_1$-$C_4$ alkyl or $C_1$-$C_4$ alkoxy.

$R_3$, $R_4$ and $R_5$ are independently H, $CH_3$, $CF_3$ or $CH_2CH_3$;

$R_6$ and $R_7$ are independently $C_1$-$C_8$ alkyl; or $R_6$ and $R_7$ can be taken together as piperidino, morpholino, pyrrolidino, hexamethyleneimino, heptamethyleneimino, wherein these groups are optionally substituted by 1 or 2 of the following: $C_1$-$C_4$ alkyl groups or phenyl ings substituted by hydrogen or 1 or 2 halogen, $C_1$-$C_4$ alkyl, or $C_1$ or $C_4$ alkoxy groups.

X is O or S;

Y is O or S;

Z is $C_1$-$C_4$ unbranched alkylene optionally substituted by 1-2 $CH_3$, $CF_3$ or $CH_2CH_3$ groups;

m is O or 1; and

their agriculturally suitable salts.

In the above definitions, the term "alkyl," used either alone or in compound words such as "alkoxyalkyl", denotes straight chain or branched alkyl, e.g., methyl, ethyl, n-propyl, isopropyl or the different butyl isomers.

Alkoxy denotes e.g., methoxy, ethoxy, n-propoxy, isopropoxy and the different butoxy isomers.

Alkenyl denotes straight chain or branched alkenes, e.g., vinyl, 1-propenyl, 2-propenyl, 3-propenyl and the different butenyl isomers.

Alkynyl denotes straight chain or branched alkynes, e.g., ethynyl, 1-propynyl, 2-propynyl and the different butynyl isomers.

The total number of carbon atoms in a substituent group is indicated by the $C_i$-$C_j$ prefix where i and j are numbers from 1 to 8. For example, $C_3$-$C_4$ alkenyl would designate propenyl through butenyl.

Preferred for the reason of more efficient control of fungal diseases are:

1. The method wherein

$R_1$ and $R_2$ are located at the 6 and 7 positions of the benzene ring; and

$R_3$, $R_4$ and $R_5$ are independently H or $CH_3$.

2. The method of Preferred 1 wherein

$R_1$ is H, $R_2$ is $C_1$-$C_4$ alkyl, $C_1$-$C_4$ alkoxy, $C_1$-$C_4$ alkoxyalkyl or halogen; and

$R_6$ and $R_7$ are taken together as piperidino, morpholino or pyrrolidino optionally substituted by phenyl or 1-2 $C_1$-$C_2$ alkyl groups.

3. The method of Preferred 2 wherein

Z is $C_1$-$C_3$ unbranched alkylene optionally substituted by a $CH_3$ group.

4. The method of Preferred 3 wherein

$R_6$ and $R_7$ are taken together as piperidino, morpholino, pyrrolidino, 2,6-dimethylmorpholino, 3,5-dimethylpiperidino and 4-phenylpiperidino;

$R_2$ is $C_2$-$C_4$ alkyl or $C_2$-$C_4$ alkoxyalkyl; X and Y are O.

Specifically preferred for the reason of most efficient control of fungal diseases is the method wherein the compound of Formula I is selected from the group consisting of:

(1) 1-[2-[6-and-7-(1,1-dimethylethyl)-2,3-dihydro-1,4-benzodioxan-2-yl]ethyl]-trans-3,5-dimethylpiperidine;

(2) 1-[2-[6-and-7-(1,1-dimethylethyl)-2,3-dihydro-1,4-benzodioxan-2-yl-ethyl]-cis-3,5-dimethylpiperidine;

(3) 1-[[2,3-dihydro-6-and-7-(1,1-dimethylethyl)-1,4-benzodioxan-2-yl]methyl]-cis-3,5-dimethylpiperidine;

(4) 1-[[6-(1,1-dimethylethyl)-2,3-dihydro-1,4-benzodioxan-2-yl]methyl]piperidine;

(5) 1-[2-[6-and-7-(1,1-dimethylethyl)-2,3-dihydro-1,4-benzodioxan-2-yl]ethyl]-cis-3,5-dimethylpiperidine

hydrochloride;

(6) 1-[[2,3-dihydro-6-and-7-(1,1-dimethylethyl)-1,4-benzodioxan-2-yl]methyl]-cis-3,5-dimethylpiperidine hydrochloride;

(7) cis-1-[[6- and 7-(1,1-dimethylethyl)-2,3-dihydro-3-methyl-1,4-benzodioxan-2-yl]methyl]-cis-3,5-dimethylpiperidine;

and

(8) 4-[2-[6-and 7-(1,1-dimethylethyl)-2,3-dihydro-1,4-benzodioxan-2-yl]ethyl]-cis-2,6-dimethylmorpholine.

This invention also comprises compounds of formula II and agriculturally suitable compositions containing them

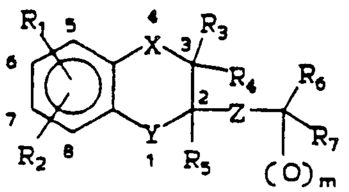

II

wherein

$R_1$ and $R_2$ are independently H; halogen; $C_1$-$C_8$ alkyl; $C_2$-$C_8$ alkenyl; $C_2$-$C_8$ alkynyl; $C_1$-$C_8$ alkoxy; $C_1$-$C_5$ alkyl substituted with 1-3 F, Cl or Br or $C_2$-$C_4$ alkoxyalkyl; $Si(CH_3)_3$; thioalkyl; or phenyl substituted with one or two of the following: halogen, $C_1$-$C_4$ alkyl or $C_1$-$C_4$ alkoxy;

$R_3$, $R_4$ and $R_5$ are independently H, $CH_3$, $CF_3$ or $CH_2CH_3$;

$R_6$ and $R_7$ are independently $C_1$-$C_8$ alkyl; or $R_6$ and $R_7$ can be taken together as piperidino, morpholino, pyrrolidino, hexamethyleneimino, heptamethyleneimino, wherein these groups are optionally substituted by 1 or 2 are of the following: $C_1$-$C_4$ alkyl, or phenyl rings substituted by hydrogen or 1 or 2 halogen, $C_1$-$C_4$ alkyl, or $C_1$ or $C_4$ alkoxy groups.

X is O or S;

Y is O or S;

Z is $C_1$-$C_4$ unbranched alkylene optionally substituted by 1-2 $CH_3$, $CF_3$ or $CH_2CH_3$ groups;

m is O or 1; and

their agriculturally suitable salts provided that

(a) when X and Y are both O, and Z is unbranched alkylene, then $R_6$ and $R_7$ are taken together as piperidino substituted at the 2, 3, 5 or 6 positions, or as substituted morpholine;

(b) when X and Y are both O, Z is $CHCH_3$, and $R_1$ and $R_2$ are both H, then $R_6$ and $R_7$ are taken together as substituted piperidino or morpholino.

Preferred for the reasons of increased ease of synthesis and/or greater fungicidal efficacy are:

1. The method wherein

$R_1$ and $R_2$ are located at the 6 and 7 positions of the benzene ring; and

$R_3$, $R_4$ and $R_5$ are independently H or $CH_3$.

2. The method of Preferred 1 wherein

$R_1$ is H, $R_2$ is $C_1$-$C_4$ alkyl, $C_1$-$C_4$ alkoxy, $C_1$-$C_4$ alkoxyalkyl or halogen; and

$R_6$ and $R_7$ are taken together as piperidino, morpholino or pyrrolidino optionally substituted by phenyl or 1-2 $C_1$-$C_2$ alkyl groups.

3. The method of Preferred 2 wherein

Z is $C_1$-$C_3$ unbranched alkylene optionally substituted by a $CH_3$ group.

4. The method of Preferred 3 wherein

$R_6$ and $R_7$ are taken together as piperidino, morpholino, pyrrolidino, 2,6-dimethylmorpholino, 3,5-dimethylpiperidino and 4-phenylpiperidino;

$R_2$ is $C_2$-$C_4$ alkyl or $C_2$-$C_4$ alkoxyalkyl; X and Y are O.

Specifically preferred for the reason of greatest ease of synthesis and/or greatest fungicidal efficacy are:

(1) 1-[2-[6-and-7-(1,1-dimethylethyl)-2,3-dihydro-1,4-benzodioxan-2-yl]ethyl]-trans-3,5-dimethylpiperidine;

(2) 1-[2-[6-and-7-(1,1-dimethylethyl)-2,3-dihydro-1,4-benzodioxan-2-yl-ethyl]-cis-3,5-dimethylpiperidine;

(3) 1-[[2,3-dihydro-6-and-7-(1,1-dimethylethyl)-1,4-benzodioxan-2-yl]methyl]-cis-3,5-dimethylpiperidine;

(4) 1-[[6-(1,1-dimethylethyl)-2,3-dihydro-1,4-benzodioxan-2-yl]methyl]piperidine;

(5) 1-[2-[6-and-7-(1,1-dimethylethyl)-2,3-dihydro-1,4-benzodioxan-2-yl]ethyl]-cis-3,5-dimethylpiperidine hydrochloride;

(6) 1-[[2,3-dihydro-6-and-7-(1,1-dimethylethyl)-1,4-benzodioxan-2-yl]methyl]-cis-3,5-dimethylpiperidine hydrochloride;

(7) cis-1-[[6- and 7-(1,1-dimethylethyl)-2,3-dihydro-3-methyl-1,4-benzodioxan-2-yl]methyl]-cis-3,5-dimethylpiperidine;

and

(8) 4-[2-[6-and 7-(1,1-dimethylethyl)-2,3-dihydro-1,4-benzodioxan-2-yl]ethyl]-cis-2,6-dimethylmorpholine.

## DETAILED DESCRIPTION OF THE INVENTION

Compounds of Formula I (m = O) can be prepared by the displacement of a leaving group L in II with an amine III. Typical leaving groups include halides and sulfonates. The reaction is run neat with one to three equivalents of the amine with or without an additional base such as potassium carbonate at temperatures between 25 and 150° C. In addition, a solvent such as DMF may also be used.

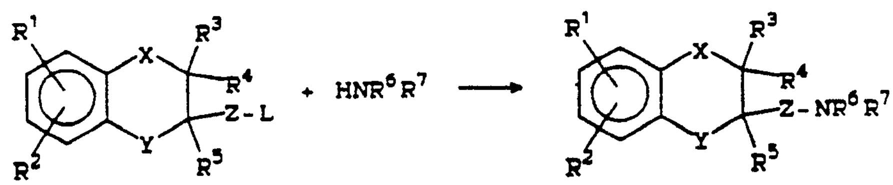


II III I

The compounds IIa where X-Z, $R_1$-$R_7$ are as defined above and L is a sulfonate can be prepared from an alcohol IV and sulfonyl chloride (e.g., toluene sulfonyl chloride) in a solvent such as pyridine at temperatures between 0 and 25° C.

Compounds IIb where L is a halogen can be prepared from an alcohol IV and thionyl chloride using the standard conditions known to one familiar with the art. The iodide can be prepared from the chloride using sodium iodide/acetone.

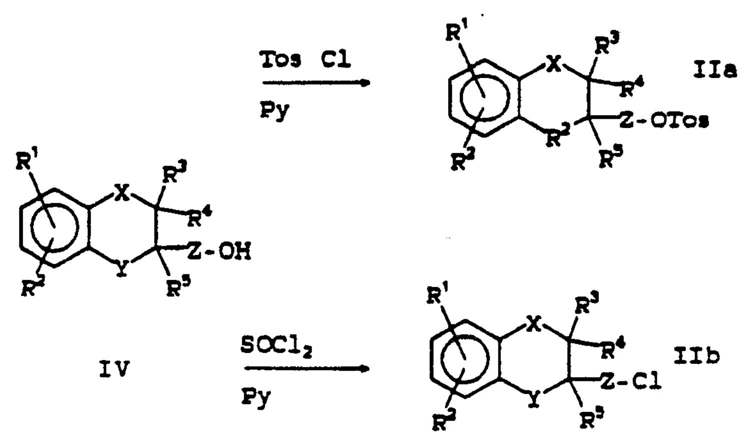


IV IIa IIb

The alcohols IVa where X=Y= O, S and Z=$CH_2$ can be prepared by heating at 30 to 100° C substituted catechols of general Formula V and epichlorohydrin VI in the presence of an aqueous base such

as potassium or sodium hydroxide at pressures between atmospheric and 100 psi as taught in U.S. 2,056,046. A mixture of regioisomers IVa is obtained using this method.

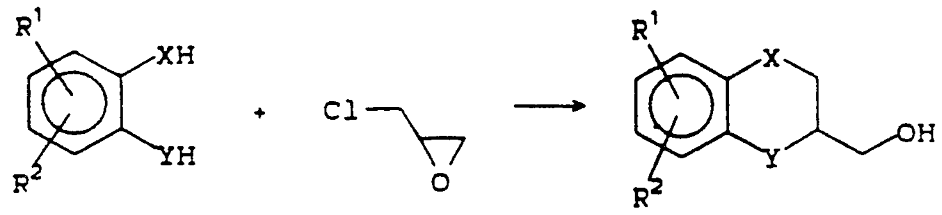

V VI IVa

The regiospecific isomers IVa (X, Y = O) can be prepared from substituted 2-hydroxyacetophenones VII. Treatment of VII with epichlorohydrin (VI) in a protic solvent such as aqueous ethanol at reflux gives epoxides VIII. Oxidation with a peracid such as m-chloroperoxybenzoic acid (m-CPBA) in a halogenated solvent or ethyl acetate at temperatures between 25 and 100 °C forms the esters IX. Subsequent aqueous base hydrolysis provides the alcohols as taught by R. Henning, J. Med. Chem., 1987, 30, 814-819.

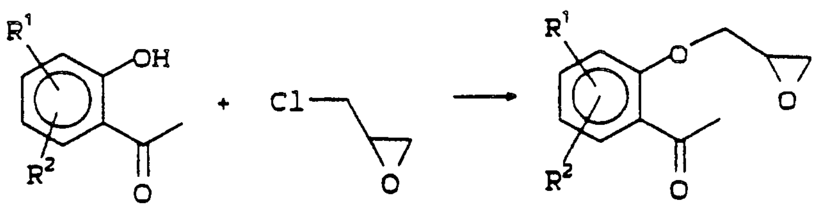

VII VI VIII

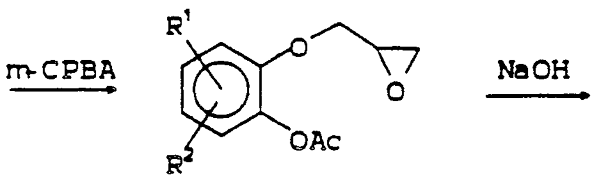


IVa

IX

Chain extended alcohols such as IVb where Z = $CH_2CH_2$, $CH(CH_3)CH_2$, or $CH(CH_2CH_3)CH_2$ can be prepared by the reduction of the corresponding esters X (where $R_8$ = H, Me, Et) with a suitable reducing agent, for example lithium aluminum hydride in a solvent such as tetrahydrofuran. The esters X are prepared from substituted catechols or thiocatechols of formula V and a (un)substituted ethyl bromocrotonate in acetone or acetonitrile with a base such as potassium carbonate as taught by G. Cerioni, J. Heterocyclic Chem., 23, 1815 (1886). This method provides a mixture of regioisomers.

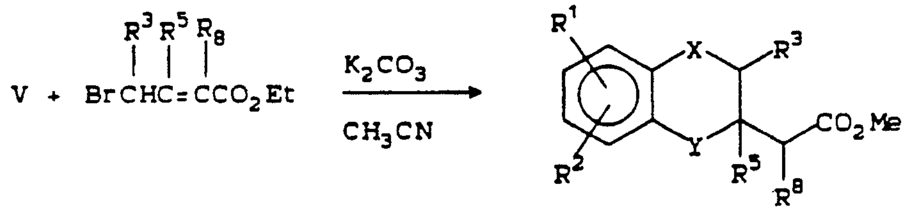


X

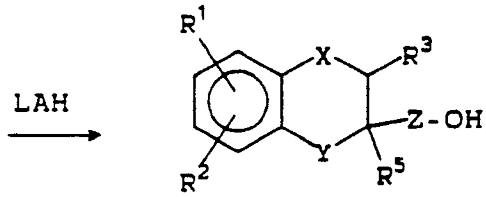


IVb

Chain extended esters X can also be obtained from aqueous acid hydrolysis of the corresponding nitriles XI. This reaction is conveniently carried out in aqueous methanolic hydrochloric acid at reflux. The nitriles XI are prepared from substituted catechols or thiocatechols of formula V and a 3,4-dibromobutyronitrile as taught in U.S. 4,104,396 and U.S. 4,129,655.

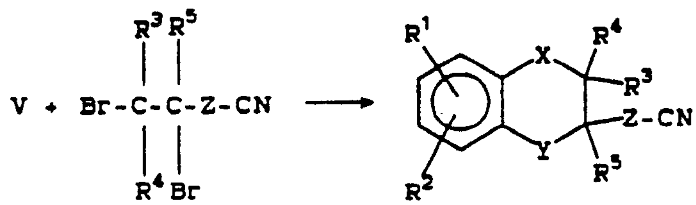


XI

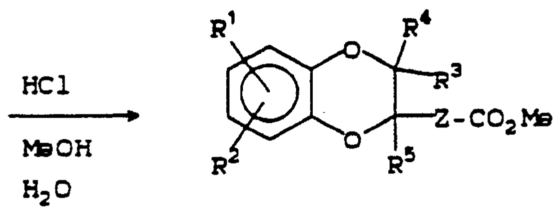


Xa

Alternatively, these same nitriles XI can be obtained from the corresponding chlorides IIa and a metal cyanide, e.g., sodium cyanide in an alcoholic solvent such as ethanol or methanol.

$$IIa \xrightarrow[EtOH]{NaCN} XI$$

In an alternate process, compounds of Formula I can be prepared by reduction of the corresponding amide XII using a reducing reagent such as lithium aluminum hydride in a convenient solvent, for example, tetrahydrofuran.

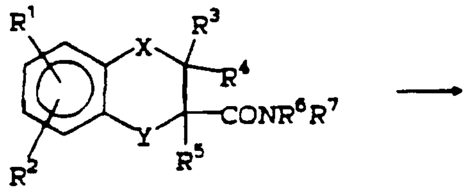

I

XII

The amides XII are prepared from the corresponding acid chlorides XIII and amine III in a solvent such as tetrahydrofuran or methylene chloride using a tertiary amine base as an acid scavenger, for example, triethylamine.

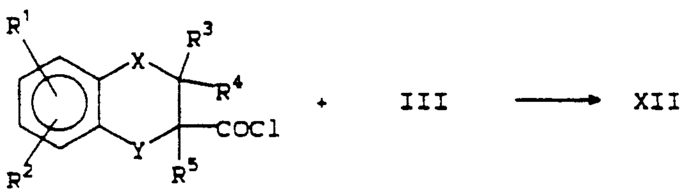

III XII

XIII

The acid chlorides XIII are prepared from the corresponding acids X using standard reaction conditions known to one skilled in the art.

The N-oxides of Formula I can be prepared from the amines by oxidation using standard reaction conditions known to those skilled in the art.

The acid salts of formula I can be prepared by treatment of the free base with the corresponding acid (e.g., hydrogen chloride) in an organic solvent such as diethyl ether.

## Example 1

### Preparation of [6-and-7-(1,1-Dimethylethyl)-2,3-dihydro-1,4-benzodioxan-2-yl]methanol

To 10.0 g (60.2 mmol) of 4-t-butylcatechol and 6.0 g of potassium hydroxide stirring in 80 mL of ethanol at room temperatures, 6.0 mL of epichlorohydrin was added. The mixture was stirred at room temperature for 30 minutes before being heated at reflux 40 minutes. The reaction was then allowed to stand overnight. Another 2.0 g of potassium hydroxide and 6.0 mL of water was added and the reaction heated at reflux 20 minutes. Excess water and 200 mL of diethyl ether was added. The ether extract was washed with water (2X), saturated sodium bicarbonate, brine, dried over magnesium sulfate, and evaporated in vacuo to give a crude oil. The title compounds, which were the main component present, were isolated as a regioisomer mixture by silica gel column chromatography (1:1 hexane/diethyl ether) whereby 6.3 g of oil were obtained. This material was used directly (as a mixture) in the next step.

## Example 2

### Preparation of [[6-and-7-(1,1-Dimethylethyl)-2,3-dihydro-1,4-benzodioxan-2-yl]methyl]tosylate

To 3.0 g (13.5 mmol) of [6-and-7-(1,1-dimethylethyl)-2,3-dihydro-1,4-benzodioxin-2-yl]methanol stirring in 15.0 mL of pyridine at about 5-10 °C, 4.0 g (21.0 mmol) of 4-toluenesulfonylchloride were added. The

reaction was allowed to reach room temperature and stirred overnight. The reaction mixture was poured into a mixture of ice water and concentrated hydrochloric acid followed by extraction with a mixture of 150 mL of diethyl ether and 50 mL of ethyl acetate. The organic layer was washed with 5% aqueous hydrochloric acid, water, brine, dried over magnesium sulfate and evaporated in vacuo to give an oil. A solid was triturated on addition of hexane and a small amount of n-butylchloride. This solid was filtered and washed well with hexane to give 3.5 g of the title compounds (regioisomer mixture), m.p. 89-96 °C.

## Example 3

### Preparation of cis and trans (+/-)-1-[[6-and-7-(1,1-Dimethylethyl)-2,3-dihydro-1,4-benzodioxan-2-yl]methyl]-3,5-dimethylpiperidine

A mixture of 2.0 g (5.3 mmol) [[6-and-7-(1,1-dimethylethyl)-2,3-dihydro-1,4-benzodioxin-2-yl]methyl]-tosylate, 6.0 mL of 3,5-dimethylpiperidine and 1.0 g of powdered potassium carbonate was heated neat with stirring at 100 °C for 15 minutes. After cooling, excess water and 100 mL of diethyl ether was added. The ether layer was separated and washed with brine, dried over magnesium sulfate, and evaporated in vacuo to give an oil which was chromatographed on silica gel (5:1 followed by 3:1 hexane/diethyl ether) to afford 250 mg of the minor trans(dimethylpiperidine) isomer and 1.3 g of the major cis(dimethylpiperidine) isomer of the title compounds. Both components were oils and were about 1:1 mixtures of the above t-butyldioxane regioisomers.

## Example 4

### Preparation of 1-[[6-and 7-(1,1-Dimethylethyl)-2,3-dihydro-1,4-benzodioxan-2-yl]methyl]-4-phenylpiperidine

A mixture of 2.0 g (5.3 mmol) [[6-and-7-(1,1-Dimethyl-ethyl)-2,3-dihydro-1,4-benzodioxin-2-yl]methyl]-tosylate, 2.0 g (12.4 mmol) of 4-phenylpiperidine and 1.5 g of powdered potassium carbonate was heated in 6.0 mL of dimethylformamide at about 90 °C for 15 minutes. Excess water and 150 mL of diethyl ether were added. The ether layer was washed with water (2X), brine, dried over magnesium sulfate, and evaporated in vacuo to give an oil which was chromatographed on silica gel (1:1 hexane/ether) to give 1.5 g of the title compounds, isolated as an oil. This material was about a 1:1 mixture of the above t-butyldioxane regioisomers.

## Example 5

### Preparation of [[6-(1,1-Dimethylethyl)-2,3-dihydro-1,4-benzodioxan-2-yl]methyl]tosylate

By the synthetic procedure reported by R. Henning et al. in J. Med. Chem., Vol. 30, pp. 814-819 (1987), a sample of [6-(1,1-dimethylethyl)-2,3-dihydro-1,4-benzodioxin-2-yl]methanol was prepared regioselectivity.

To 1.5 g (6.8 mmol) of [6-(1,1-dimethylethyl)-2,3-dihydro-1,4-benzodioxin-2-yl]methanol stirring in 10 mL of pyridine at about 0 °C, 2.0 g (10.5 mmol) of 4-toluenesulfonylchloride was added. The reaction was allowed to reach room temperature and stirred overnight. The mixture was poured into ice water containing some concentrated hydrochloric acid and the aqueous mixture extracted with 150 mL of diethyl ether. The ether extract was washed with 5% HCl, water, brine, dried over magnesium sulfate, and evaporated in vacuo to give an oil to which hexane was added. Trituration and filtering gave 2.2 g of the title compound, isolated as a white solid (m.p. 96-98 °C).

Example 6

Preparation of 1-[[6-(1,1-Dimethylethyl)-2,3-dihydro-1,4-benzodioxan-2-yl]methyl]piperidine

A mixture of 2.0 g (5.3 mmol) [[6-(1,1-dimethylethyl)-2,3-dihydro-1,4-benzodioxin-2-yl]methyl]tosylate, 8.0 mL of piperidine, and 1.0 g of powdered potassium carbonate was heated with stirring neat at reflux, with a significant amount of foaming, for 15 minutes. After cooling, excess water and brine were added along with 100 mL of diethyl ether. The ether layer was washed with brine, dried over magnesium sulfate, and evaporated in vacuo to give an oil which was chromatographed on silica gel (2:1 hexane/ether) to give 1.4 g of the title compound, isolated as an oil.

Example 7

Preparation of cis and trans (+/-)[[6-(1,1-Dimethylethyl)-2,3-dihydro-1,4-benzodioxan-2-yl]methyl]-3,5-dimethylpiperidine

A mixture of 3.0 g (8.0 mmol) [[6-(1,1-dimethylethyl)-2,3-dihydro-1,4-benzodioxin-2-yl]methyl]tosylate, 5.0 mL dimethylpiperidine, and 1.5 g powdered potassium carbonate was heated neat with stirring at 90 °C for 15 minutes. After cooling, excess water and brine were added along with 150 mL of diethyl ether. The ether extract was washed with brine, dried over magnesium sulfate, and evaporated in vacuo to give an oil which was chromatographed on silica gel (5:1 followed by 3:1 hexane/ether) to give 0.5 g of the minor trans-(dimethylpiperidine) isomer and 1.2 g of the major cis(dimethylpiperidine) isomer of the title compound. Both of these isomers were isolated as oils.

Example 8

Preparation of Ethyl [6-and-7-(1,1-dimethylethyl)-2,3-dihydro-1,4-benzodioxan-2-yl]acetate

A mixture of 15.0 g (90.4 mmol) of 4-t-butylcatechol, 15.0 mL of ethyl 4-bromocrotonate and 10.0 g of powdered potassium carbonate was heated in 150 mL of acetonitrile at reflux 2 hours. Excess water and 300 mL of diethyl ether were added. The ether layer was separated and washed with water (2X), brine, dried over magnesium sulfate, and evaporated in vacuo to give a crude oil. The title compound which was the main component present was isolated by silica gel column chromatography (10:1 followed by 5:1 hexane/diethyl ether). A yield of 17.0 g of oil was obtained. This material, which was a mixture of above t-butylbenzodioxane regioisomers, was used directly in subsequent preparation of the free acid.

Example 9

Preparation of [6-and-7-(1,1-Dimethylethyl)-2,3-dihydro-1,4-benzodioxan-2-yl]acetic acid

To 6.5 g (23.4 mmol) of ethyl [6-and-7-(1,1-dimethylethyl)-2,3-dihydro-1,4-benzodioxin-2-yl]acetate stirring in 75 mL methanol and 5.0 mL water, 6.0 g of 50 wt% aqueous sodium hydroxide were added and the mixture heated at reflux about 5 minutes. After cooling, the reaction mixture was acidified with 10% hydrochloric acid to about pH 1 and 150 mL of ethyl acetate was added. The ethyl acetate extract was separated and washed with water, brine, dried over magnesium sulfate, and evaporated in vacuo to give 7.3 g of the title compounds (mixture of regioisomers) which was isolated initially as an oil but gradually turned into an oily solid which was used directly in preparation of the acid chloride.

Example 10

Preparation of 1-[2-[6-and-7-(1,1-Dimethylethyl)-2,3-dihydro-1,4-benzodioxan-2-yl]ethyl]piperidine

To 7.2 g (28.5 mmol) of [6-and-7-(1,1-dimethylethyl)-2,3-dihydro-1,4-benzodioxin-2-yl]acetic acid stirring in 75 mL of benzene, 8.0 mL of thionyl chloride was added along with 2 drops of dimethylformamide. The reaction was heated at reflux 1.5 hours and then evaporated in vacuo to dryness. The residue was dissolved in 75 mL of tetrahydrofuran and at 0° C, 8.0 mL of piperidine was added dropwise to the stirred solution. A thick suspension resulted which was stirred 1 hour at room temperature. Diethyl ether (100 mL) was added to the suspension and the solid filtered and washed with additional ether. The filtrate was evaporated in vacuo to give an oily residue which was chromatographed on silica gel (2:1 followed by 3:1 diethyl ether/hexane) to give 5.0 g of the amide intermediate, isolated as a yellow oil.

To 1.4 g (4.4 mmol) of this amide intermediate stirring in 30 mL of tetrahydrofuran, 0.7 g of solid lithium aluminum hydride added and the mixture stirred at room temperature overnight before heating at reflux 1.5 hours. At about 0° C, 20 mL of ethyl acetate was slowly added followed by the addition of 4.0 mL water and 3.0 mL 15% aqueous sodium hydroxide. Diethyl ether (100 mL) was then added and the mixture stirred 1 hour before filtering through celite. The filtrate was dried over magnesium sulfate and evaporated in vacuo to give an oil which was chromatographed on silica gel (5% methanol in methylene chloride) to give 1.0 g of the title compound, isolated as an oil.

Example 11

Preparation of cis and trans (+/-)-1-[2-[6-and-7-(1,1-Dimethylethyl)-2,3-dihydro-1,4-benzodioxan-2-yl]ethyl]-3,5-dimethylpiperidine

To 4.0 g (16.0 mmol) of [6-and-7-(1,1-dimethylethyl)-2,3-dihydro-1,4-benzodioxin-2-yl]acetic acid stirring in 50 mL of benzene, 2 drops of dimethylformamide along with 5.0 mL of thionylchloride were added. The reaction was heated at reflux 40 minutes, cooled, and evaporated in vacuo to dryness. The residue was dissolved in 50 mL of tetrahydrofuran and at 0° C, 5.0 mL of dimethylpiperidine was added dropwise. The suspension was stirred at room temperature overnight. Diethyl ether (100 mL) was added to the suspension to enhance precipitation of solid and the mixture filtered. The filtrate was evaporated in vacuo to give an oil which was chromatographed on silica gel (4:1 followed by 1:1 hexane/diethyl ether) to give 2.3 g of the amide intermediate, isolated as an oil.

To 2.0 g (5.8 mmol) of this amide intermediate stirring in 30 mL of tetrahydrofuran, 1.0 g of solid lithium aluminumhydride added and the mixture heated at reflux 1.5 hours. At about 0° C, 20 mL of ethyl acetate was slowly added followed by the addition of 1.0 mL of water, 2.0 mL of 15% aqueous sodium hydroxide and another 2.0 mL of water. Diethyl ether (100 mL) was added and the thick suspension stirred 1 hour before filtering through celite. The filtrate was dried over magnesium sulfate and evaporated in vacuo to give an oil which was chromatographed on silica gel (2% methanol in methylene chloride) to afford 0.3 g of the minor trans(dimethylpiperidine) isomer and 0.7 g of the major cis(dimethylpiperidine) isomer of the title compound. Both components, which were about 1:1 mixtures of the above t-butylbenzodioxane regioisomers, were oils.

Example 12

Preparation of [6- and 7-(1,1-dimethylethyl)-2,3-dihydro-1,4-benzodioxan-2-yl]-2-ethanol

To a solution of 14.0 g (50.4 mmol) of ethyl [6-and 7-(1,1-dimethylethyl)-2,3-dihydro-1,4-benzodioxan-2-yl]-acetate stirring in 250 ml of diethyl ether at 0° C, 2.0 g of lithium aluminum hydride was added portionwise. After the addition, the reaction was allowed to warm to room temperature and stirred 1 hour.

Ethyl acetate (20 ml) was added dropwise followed by the addition of 4.0 ml water, 4.0 ml 15% sodium hydroxide followed in turn with 2.0 ml of water. The resulting suspension was stirred two hours before filtering through celite. The filtrate was dried over magnesium sulfate and evaporated in vacuo to give an oil (11.5 g) which was used directly in the next step (preparation of the tosylate).

## Example 13

### Preparation of [[6- and 7-(1,1-dimethylethyl)-2,3-dihydro-1,4-benzodioxan-2-yl]-2-ethyl]tosylate

A sample of 5.0 g (26.2 mmol) of H-toluene-sulfonyl chloride was added to a solution of 5.0 g (21.2 mmol) of [6- and 7-(1,1-dimethylethyl)-2,3-dihydro-1,4-benzodioxan-2-yl]-2-ethanol stirring in 25 ml of pyridine at about 0 °C. The reaction mixture was stirred at 0-5 °C for 1.5 hours followed by stirring at ambient temperature for 0.5 hour. Diethyl ether (200 ml) was added followed by the addition of 100 ml of 5% hydrochloric acid. The ether layer was separated and washed with another 100 ml of 5% hydrochloric acid, water, brine, and dried over magnesium sulfate. Evaporating in vacuo gave an oily residue from which 5.5 g of solid was triturated with hexane, m.p. 77-86 °C.

## Example 14

### Preparation of 1-[[6- and 7-(1,1-dimethylethyl)-2,3-dihydro-1,4-benzodioxan-2-yl]-2-ethyl]-heptamethyleneimine

A stirred mixture of 1.5 g (3.85 mmol), 0.7 g potassium carbonate, and 8.0 ml of heptamethyleneimine was heated neat at 90 °C for 2 hours. Xylene (20 ml) was added, the reaction mixture filtered, and the filtrate evaporated in vacuo to give an oily residue. Silica gel column chromatography (3:1 followed by 2:1 hexane/ethyl acetate) afforded 1.1 g of the desired product was isolated as an oil.

## Example 15

### Preparation of cis(±)-1-[[6- and 7-(1,1-dimethylethyl)-2,3-dihydro-1,4-benzodioxan-2-yl]methyl]-3,5-dimethylpiperidine hydrochloride

To 1.5 g (4.7 mmol) of the free base of the title compound, in 15 ml of diethyl ester, gaseous hydrogen chloride was bubbled in for approximately 2 minutes. The thick white precipitate which resulted was filtered and washed with diethyl ether to give 1.4 g of the desired hydrochloride salt, m.p. 205-217 °C.

Using the general procedures outlined above and those of Examples 1-11, the compounds of Tables 1 and 2 can be prepared.

TABLE 1

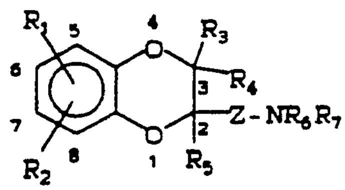

| $R_1$ | $R_2$ | $R_3$ | $R_4$ | $R_5$ | Z | $NR_6R_7$ |
|---|---|---|---|---|---|---|
| H | H | H | H | H | $CH_2$ | piperidino |
| H | H | H | H | H | $CH_2$ | cis-3,5-dimethylpiperidino |
| H | H | H | H | H | $CH_2$ | trans-3,5-dimethylpiperidino |
| H | H | H | H | H | $CH_2$ | cis-2,6-dimethylmorpholino |
| H | H | H | H | H | $CH_2$ | 4-phenylpiperidino |
| H | H | H | H | H | $CH_2$ | N,N-dibutylamino |
| H | H | $CH_3$ | H | H | $CH_2$ | piperidino |
| H | H | H | H | $CH_3$ | $CH_2$ | cis-3,5-dimethylpiperidino |
| H | H | $CH_3$ | $CH_3$ | H | $CH_2$ | piperidino |
| H | H | $CH_3$ | H | $CH_3$ | $CH_2$ | 4-phenylpiperidino |
| 6-$CH_3$ | H | H | H | H | $CH_2$ | piperidino |
| 6-$CH_3$ | H | H | H | H | $CH_2$ | cis-3,5-dimethylpiperidino |
| H | 7-$CH_3$ | H | H | H | $CH_2$ | trans-3,5-dimethylpiperidino |
| H | 7-$CH_3$ | H | H | H | $CH_2$ | 2,6-dimethylmorpholino |
| H | 7-$CH_3$ | H | H | H | $CH_2$ | 4-phenylpiperidino |
| OMe | H | H | H | H | $CH_2$ | cis-3,5-dimethylpiperidino |
| OMe | H | H | H | H | $CH_2$ | 4-phenylpiperidino |
| H | 7-OMe | H | H | H | $CH_2$ | cis-3,5-dimethylpiperidino |
| H | 7-OMe | H | H | H | $CH_2$ | 4-phenylpiperidino |
| 6-$C_2H_5$ | H | H | H | H | $CH_2$ | cis-3,5-dimethylpiperidino |
| 6-$C_2H_5$ | H | H | H | H | $CH_2$ | 4-phenylpiperidino |

| $R_1$ | $R_2$ | $R_3$ | $R_4$ | $R_5$ | Z | $NR_6R_7$ |
|---|---|---|---|---|---|---|
| H | 7-$C_2H_5$ | H | H | H | $CH_2$ | cis-3,5-dimethylpiperidino |
| H | 7-$C_2H_5$ | H | H | H | $CH_2$ | 4-phenylpiperidino |
| 6-t-Bu | H | H | H | H | $CH_2$ | piperidino |
| 6-t-Bu | H | H | H | H | $CH_2$ | 4-methylpiperidino |
| 6-t-Bu | H | H | H | H | $CH_2$ | 4-phenylpiperidino |
| 6-t-Bu | H | H | H | H | $CH_2$ | 3,3-dimethylpiperidino |
| 6-t-Bu | H | H | H | H | $CH_2$ | cis-3,5-dimethylpiperidino |
| 6-t-Bu | H | H | H | H | $CH_2$ | trans-3,5-dimethylpiperidino |
| 6-t-Bu | H | H | H | H | $CH_2$ | morpholino |
| 6-t-Bu | H | H | H | H | $CH_2$ | cis-2,6-dimethylmorpholino |
| 6-t-Bu | H | H | H | H | $CH_2$ | pyrrolidino |
| H | 7-t-Bu | $CH_3$ | $CH_3$ | H | $CH_2$ | piperidino |
| 6-t-Bu | H | $CH_3$ | $CH_3$ | H | $CH_2$ | piperidino |
| H | 7-t-Bu | H | H | H | $CH_2CH_2CH_2$ | cis-3,5-dimethylpiperidino |
| 6-t-Bu | H | H | H | H | $CH_2CH_2CH_2$ | trans-3,5-dimethylpiperidino |
| 6-Cl | 7-Cl | H | H | H | $CH_2$ | cis-3,5-dimethylpiperidino |
| 5-Cl | H | H | H | H | $CH_2$ | cis-3,5-dimethylpiperidino |
| 5-OMe | H | H | H | H | $CH_2$ | 4-phenylpiperidino |
| 5-Cl | 6-Cl | H | H | H | $CH_2$ | 4-phenylpiperidino |
| 5-n-butyl | H | H | H | H | $CH_2$ | cis-3,5-dimethylpiperidino |
| 6,7-$(CH_2)_4$- | | H | H | H | $CH_2$ | cis-3,5-dimethylpiperidino |
| 5-t-butyl | | H | H | H | $CH_2$ | 4-phenylpiperidino |
| 6-t-Bu | H | H | H | H | $CH_2$ | N,N-dipropylamino |
| H | 7-t-Bu | H | H | H | $CH_2$ | piperidino |
| H | 7-t-Bu | H | H | H | $CH_2$ | 4-methylpiperidino |
| H | 7-t-Bu | H | H | H | $CH_2$ | 4-phenylpiperidino |
| H | 7-t-Bu | H | H | H | $CH_2$ | 3,3-dimethylpiperidino |
| H | 7-t-Bu | H | H | H | $CH_2$ | cis-3,5-dimethylpiperidino |
| H | 7-t-Bu | H | H | H | $CH_2$ | trans-3,5-dimethylpiperidino |
| H | 7-t-Bu | H | H | H | $CH_2$ | morpholino |
| H | 7-t-Bu | H | H | H | $CH_2$ | cis-2,6-dimethylmorpholino |

| $R_1$ | $R_2$ | $R_3$ | $R_4$ | $R_5$ | Z | $NR_6R_7$ |
|---|---|---|---|---|---|---|
| H | 7-t-Bu | H | H | H | $CH_2$ | pyrrolidino |
| H | 7-t-Bu | H | H | H | $CH_2$ | N,N-dipropylamino |
| 6-i-Pr | H | H | H | H | $CH_2$ | cis-3,5-dimethylpiperidino |
| 6-i-Pr | H | H | H | H | $CH_2$ | trans-3,5-dimethylpiperidino |
| 6-i-Pr | H | H | H | H | $CH_2$ | cis-2,6-dimethylmorpholino |
| H | 7-i-Pr | H | H | H | $CH_2$ | cis-3,5-dimethylpiperidino |
| H | 7-i-Pr | H | H | H | $CH_2$ | trans-3,5-dimethylpiperidino |
| 6-F | H | H | H | H | $CH_2$ | cis-3,5-dimethylpiperidino |
| H | 7-F | H | H | H | $CH_2$ | cis-2,6-dimethylmorpholino |
| 6-Cl | H | H | H | H | $CH_2$ | piperidino |
| H | 7-Cl | H | H | H | $CH_2$ | cis-3,5-dimethylpiperidino |
| 6-$CH_3$ | 7-$CH_3$ | H | H | H | $CH_2$ | piperidino |
| 6-OMe | H | H | H | H | $CH_2CH_2$ | cis-3,5-dimethylpiperidino |
| 6-OMe | H | H | H | H | $CH_2CH_2$ | 4-phenylpiperidino |
| H | 7-OMe | H | H | H | $CH_2CH_2$ | cis-3,5-dimethylpiperidino |
| H | 7-OMe | H | H | H | $CH_2CH_2$ | 4-phenylpiperidino |
| 6-OMe | 7-OMe | H | H | H | $CH_2CH_2$ | 4-phenylpiperidino |
| 6-F | H | H | H | H | $CH_2CH_2$ | cis-3,5-dimethylpiperidino |
| H | F | H | H | H | $CH_2CH_2$ | cis-2,6-dimethylmorpholino |
| 6-Cl | H | H | H | H | $CH_2CH_2$ | cis-3,5-dimethylpiperidino |
| H | 7-Cl | H | H | H | $CH_2CH_2$ | cis-2,6-dimethylmorpholino |
| 6-OMe | H | H | H | H | $CH_2CH_2CH_2$ | cis-3,5-dimethylpiperidino |
| 6-OMe | H | H | H | H | $CH_2CH_2CH_2$ | 4-phenylpiperidino |
| H | 7-OMe | H | H | H | $CH_2CH_2CH_2$ | cis-3,5-dimethylpiperidino |
| H | 7-OMe | H | H | H | $CH_2CH_2CH_2$ | 4-phenylpiperidino |
| 6-t-Bu | H | H | H | H | $CH_2CH_2$ | piperidino |
| 6-t-Bu | H | H | H | H | $CH_2CH_2$ | cis-3,5-dimethylpiperidino |
| 6-t-Bu | H | H | H | H | $CH_2CH_2$ | trans-3,5-dimethylpiperidino |
| 6-t-Bu | H | H | H | H | $CH_2CH_2$ | cis-2,6-dimethylmorpholino |
| 6-t-Bu | H | H | H | H | $CH_2CH_2$ | 4-phenylpiperidino |
| 6-t-Bu | H | H | H | H | $CH_2CH_2$ | pyrrolidino |

| $R_1$ | $R_2$ | $R_3$ | $R_4$ | $R_5$ | Z | $NR_6R_7$ |
|---|---|---|---|---|---|---|
| H | 7-t-Bu | H | H | H | $CH_2CH_2$ | piperidino |
| H | 7-t-Bu | H | H | H | $CH_2CH_2$ | cis-3,5-dimethylpiperidino |
| H | 7-t-Bu | H | H | H | $CH_2CH_2$ | trans-3,5-dimethylpiperidino |
| H | 7-y-Bu | H | H | H | $CH(CH_3)$ | cis-3,5-dimethylpiperidino |
| 6-t-Bu | H | H | H | | $CH(CH_3)$ | cis-3,5-dimethylpiperidino |
| H | 7-t-Bu | $CH_3$ | H | H | $CH_2CH_2$ | cis-3,5-dimethylpiperidino |
| 6-t-Bu | H | $CH_3$ | H | H | $CH_2CH_2$ | cis-3,5-dimethylpiperidino |
| H | 7-t-Bu | $CH_3$ | H | H | $CH_2CH_2$ | piperidino |
| 6-t-Bu | H | $CH_3$ | $CH_3$ | H | $CH_2$ | piperidino |
| H | 7-t-Bu | $CH_3$ | $CH_3$ | H | $CH_2$ | piperidino |
| 6-t-Bu | H | $CH_3$ | $CH_3$ | H | $CH_2$ | piperidino |
| 6-isoamyl | H | H | H | H | $CH_2$ | cis-3,5-dimethylpiperidino |
| H | 7-iso-amyl | H | H | H | $CH_2$ | cis-3,5-dimethylpiperidino |
| H | 7-t-Bu | H | H | H | $CH_2CH_2$ | cis-2,6-dimethylmorpholino |
| H | 7-t-Bu | H | H | H | $CH_2CH_2$ | 4-phenylpiperidino |
| H | 7-t-Bu | H | H | H | $CH_2CH_2$ | pyrrolidino |
| H | 7-t-Bu | H | H | H | $CH_2CH_2CH_2$ | cis-2,6-dimethylmorpholino |
| H | 7-t-Bu | H | H | H | $CH_2CH_2CH_2$ | piperidino |
| H | 7-t-Bu | H | H | H | $CH_2CH_2CH_2$ | morpholino |
| H | 7-t-Bu | H | H | H | $CH_2CH(CH_3)CH_2$ | piperidino |
| H | 7-t-Bu | H | H | H | $CH_2CH_2$ | $N(CH_3)_2$ |
| H | 7-t-Bu | H | H | H | $CH_2$ | $N(CH_2CH_3)_2$ |
| 6-t-Bu | H | H | H | H | $CH_2CH_2CH_2$ | piperidino |
| H | 7-t-Bu | $CH_3$ | H | H | $CH_2$ | cis-3,5-dimethylpiperidino |
| H | 7-t-Bu | $CH_3$ | H | H | $CH_2$ | piperidino |
| 6-t-Bu | H | $CH_3$ | H | H | $CH_2$ | cis-3,5-dimethylpiperidino |
| 6-t-Bu | H | $CH_3$ | H | H | $CH_2$ | piperidino |
| H | 7-t-Bu | $CH_3$ | H | H | $CH_2$ | 4-phenylpiperidino |
| H | 7-t-Bu | $CH_3$ | H | H | $CH_2$ | 4-phenylpiperidino |
| H | 7-t-Bu | $CH_3$ | H | H | $CH_2$ | cis-2,6-dimethylmorpholino |

| $R_1$ | $R_2$ | $R_3$ | $R_4$ | $R_5$ | Z | $NR_6R_7$ |
|---|---|---|---|---|---|---|
| 6-t-Bu | H | $CH_3$ | H | H | $CH_2$ | cis-2,6-dimethylmorpholino |
| H | 7-t-Bu | $CH_3$ | $CH_3$ | H | $CH_2$ | cis-3,5-dimethylpiperidino |
| H | 7-t-Bu | $CH_3$ | $CH_3$ | H | $CH_2$ | 4-phenylpiperidino |
| H | 7-t-Bu | H | H | H | $CH_2CH_2$ | trans-2,6-dimethylmorpholino |
| 6-t-Bu | H | H | H | H | $CH_2CH_2$ | trans-2,6-dimethylmorpholino |
| H | 7-t-Bu | H | H | $CH_3$ | $CH_2CH_2$ | cis-3,5-dimethylpiperidino |
| H | 7-t-Bu | H | H | $CH_3$ | $CH_2CH_2$ | hexamethyleneimine |
| 6-t-Bu | H | H | H | $CH_3$ | $CH_2CH_2$ | cis-2,6-dimethylmorpholino |
| 6-t-Bu | H | H | H | $CH_3$ | $CH_2CH_2$ | cis-3,5-dimethylpiperidino |
| 6-t-Bu | H | H | H | $CH_3$ | $CH_2CH_2$ | heptamethyleneimine |
| 6-t-Bu | H | H | H | $CH_3$ | $CH_2CH_2$ | trans-3,5-dimethylpiperidino |
| H | 7-t-Bu | H | H | $CH_3$ | $CH_2CH_2$ | trans-3,5-dimethylpiperidino |
| H | 7-t-Bu | H | H | H | $CH_2CH_2$ | heptamethyleneimino |
| 6-t-Bu | H | H | H | $CH_3$ | $CH_2CH_2$ | heptamethyleneimino |
| 6-t-Bu | H | H | H | $CH_3$ | $CH_2CH_2$ | hexamethyleneimino |
| H | 7-t-Bu | H | H | $CH_3$ | $CH_2CH_2$ | heptamethyleneimino |
| H | 7-t-Bu | H | H | H | $CH_2CH_2$ | hexamethyleneimino |
| 6-t-Bu | H | H | H | H | $CH_2CH_2$ | hexamethyleneimino |
| 6-t-Bu | H | H | H | H | $CH_2CH_2$ | trans-2,6-diemthylmorpholino |
| H | 7-t-Bu | H | H | H | $CH_2CH_2$ | trans-2,6-dimethylmorpholino |
| 6-t-Bu | H | H | H | $CH_3$ | $CH_2$ | cis-3,5-dimethylpiperidino |
| H | 7-t-Bu | H | H | $CH_3$ | $CH_2$ | piperidino |
| 6-t-Bu | H | H | H | $CH_3$ | $CH_2$ | piperidino |
| H | 7-t-Bu | H | H | $CH_3$ | $CH_2$ | 4-phenylpiperidino |
| H | 7-t-Bu | H | H | $CH_3$ | $CH_2$ | 4-phenylpiperidino |
| H | 7-t-Bu | H | H | H | $CH(CH_3)CH_2$ | cis-3,5-dimethylpiperidino |
| 6-t-Bu | H | H | H | H | $CH(CH_3)CH_2$ | cis-3,5-dimethylpiperidino |
| H | 8-n-Bu | H | H | H | $CH_2$ | 4-phenylpiperidino |
| 6-Br | H | H | H | H | $CH_2$ | cis-3,5-dimethylpiperidino |
| H | 7-Br | H | H | H | $CH_2$ | cis-3,5-dimethylpiperidino |
| 6-Br | H | H | H | H | $CH_2CH_2$ | cis-3,5-dimethylpiperidino |

| $R_1$ | $R_2$ | $R_3$ | $R_4$ | $R_5$ | Z | $NR_6R_7$ |
|---|---|---|---|---|---|---|
| 6-$C_6H_5$ | H | H | H | H | $CH_2$ | cis-3,5-dimethylpiperidino |
| H | 7-$C_6H_5$ | H | H | H | $CH_2$ | piperidino |
| 6-$CF_2H$ | H | H | H | H | $CH_2$ | cis-3,5-dimethylpiperidino |
| H | 7-$Si(CH_3)_3$ | H | H | H | $CH_2$ | cis-3,5-dimethylpiperidino |
| 6-$SCH_3$ | H | H | H | H | $CH_2$ | cis-3,5-dimethylpiperidino |
| H | 7-$C(CH_3)_2$ $CH_2(CH_3)_3$ | H | H | H | $CH_2$ | piperidino |
| 6-O-t-Bu | H | H | H | H | $CH_2$ | cis-3,5-dimethylpiperidino |
| H | 7-S-*t*-Bu | H | H | H | $CH_2$ | cis-3,5-dimethylpiperidino |

TABLE 2

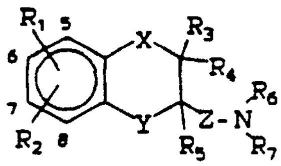

| $R_1$ | $R_2$ | $R_3$ | $R_4$ | $R_5$ | X | Y | Z | $NR_6R_7$ |
|---|---|---|---|---|---|---|---|---|
| H | H | H | H | H | O | S | $CH_2CH_2$ | cis-2,6-dimethyl-morpholino |
| 6-t-Bu | H | Me | H | H | O | S | $CH_2$ | cis-3,5-dimethyl-piperidino |
| 6-t-Bu | H | H | H | H | S | O | $CH_2CH_2$ | cis-2,6-dimethyl-morpholino |
| H | Me | H | H | H | O | S | $CH_2CH_2$ | piperidino |
| 6-t-Bu | H | H | H | H | O | S | $CH_2CH_2$ | pyrrolidino |
| H | 7-t-Bu | H | H | H | O | S | $CH_2CH_2$ | piperidino |
| H | 7-t-Bu | $CH_3$ | H | H | O | S | $CH_2$ | cis-2,6-dimethyl-morpholino |
| H | 7-t-Bu | H | H | H | O | S | $CH_2$ | cis-2,6-dimethyl-morpholino |
| 6-OMe | H | H | H | H | O | S | $CH_2CH_2$ | 4-phenyl-piperidino |
| 6-OMe | H | H | H | H | O | S | $CH_2CH_2CH_2$ | piperidino |
| H | 7-OMe | H | H | H | O | S | $CH_2CH_2$ | 4-phenyl-piperidino |
| H | 7-Cl | H | H | H | O | S | $CH_2CH_2$ | cis-3,5-dimethyl-piperidino |

| $R_1$ | $R_2$ | $R_3$ | $R_4$ | $R_5$ | X | Y | Z | $NR_6R_7$ |
|---|---|---|---|---|---|---|---|---|
| H | 7-Me | H | H | H | O | S | $CH_2$ | 4-phenyl-piperidino |
| H | 7-t-Bu | H | H | H | O | S | $CH_2CH_2$ | cis-2,6-dimethyl-morpholino |
| 6-t-Bu | H | H | H | H | O | S | $CH_2CH_2$ | morpholino |
| H | H | H | H | H | S | O | $CH_2CH_2$ | cis-3,5-dimethyl-piperidino |
| H | H | H | H | Me | S | O | $CH_2CH_2$ | cis-2,6-dimethyl-morpholino |
| 6-t-Bu | H | H | H | H | S | O | $CH_2CH_2$ | piperidino |
| H | 7-t-Bu | H | H | H | S | O | $CH_2CH_2$ | piperidino |
| 6-t-Bu | H | $CH_3$ | H | H | S | O | $CH_2$ | cis-3,5-dimethyl-piperidino |
| 6-t-Bu | H | $CH_3$ | H | H | O | S | $CH_2$ | cis-3,5-dimethyl-piperidino |
| 6-t-Bu | H | H | H | H | O | S | $CH_2$ | cis-3,5-dimethyl-piperidino |
| 6-t-Bu | H | H | H | H | S | O | $CH_2$ | cis-3,5-dimethyl-piperidino |
| 6-t-Bu | H | H | H | H | S | O | $CH_2CH_2$ | trans-3,5-dimethyl-piperidino |
| H | 7-t-Bu | H | H | H | O | S | $CH_2CH_2$ | trans-3,5-dimethyl-piperidino |
| H | 7-t-Bu | H | H | H | S | O | $CH_2CH_6$ | trans-2,6-dimethyl-morpholino |
| 6-t-Bu | H | H | H | H | O | S | $CH_2$ | piperidino |
| 6-t-Bu | H | H | H | H | S | O | $CH_2$ | piperidino |
| 6-t-Bu | H | H | H | H | S | O | $CH_2CH_2$ | 4-methyl-piperidino. |

| $R_1$ | $R_2$ | $R_3$ | $R_4$ | $R_5$ | X | Y | Z | $NR_6R_7$ |
|---|---|---|---|---|---|---|---|---|
| H | 7-OMe | H | H | H | S | O | $CH_2CH_2$ | 4-methyl-piperidino |
| H | H | H | H | H | S | S | $CH_2CH_2$ | cis-2,6-morpholino |
| 6-t-Bu | H | H | H | H | S | $CH_2O$ | $CH_2CH_2$ | morpholino |
| H | 7-t-Bu | H | H | $CH_3$ | S | O | $CH_2CH_2$ | piperidino |
| 6-OMe | H | H | H | H | S | $CH_2O$ | $CH_2CH_2$ | cis-3,5-dimethyl-piperidino |
| 6-Br | H | H | H | H | S | S | $CH_2CH_2$ | 4-phenyl-piperidino |
| 6-OMe | H | H | H | H | S | O | $CH_2CH_2$ | cis-2,6-dimethyl-morpholino |
| H | 7-Cl | H | H | H | S | $CH_2O$ | $CH_2CH_2$ | cis-3,5-dimethyl-piperidino |
| H | 7-OMe | H | H | H | S | O | $CH_2CH_2$ | piperidino |
| H | H | H | H | H | S | S | $CH_2$ | 4-phenyl-piperidino |
| H | H | H | H | H | S | S | $CH_2CH_2$ | piperidino |
| 6-Me | H | H | H | H | O | S | $CH_2CH_2$ | cis-3,5-dimethyl-piperidino |
| H | 7-Me | H | H | H | O | S | $CH_2CH_2$ | 4-phenyl-piperidino |
| H | 7-F | H | H | H | O | S | $CH_2CH_2$ | 4-phenyl-piperidino |
| 6-t-Bu | H | H | H | H | O | S | $CH_2CH_2$ | piperidino |
| 6-t-Bu | H | H | H | H | O | S | $CH_2CH_2$ | cis-2,6-dimethyl-morpholino |
| H | 7-t-Bu | H | H | H | O | S | $CH_2$ | piperidino |
| H | 7-t-Bu | H | H | H | S | O | $CH_2CH_2$ | cis-2,6-dimethyl-morpholino |

| $R_1$ | $R_2$ | $R_3$ | $R_4$ | $R_5$ | X | Y | Z | $NR_6R_7$ |
|---|---|---|---|---|---|---|---|---|
| 6-OMe | H | H | H | H | O | S | $CH_2CH_2$ | cis-3,5-dimethyl-piperidino |
| H | 7-OMe | H | H | H | O | S | $CH_2CH_2$ | 4-phenyl-piperidino |
| H | H | H | H | H | O | S | $CH_2CH_2$ | 4-phenyl-piperidino |
| 6-t-Bu | H | H | H | H | S | S | $CH_2$ | 4-phenyl-piperidino |
| H | 7-t-Bu | H | H | H | O | S | $CH_2CH_2$ | 4-phenyl-piperidino |
| 6-OMe | H | H | H | H | O | S | $CH_2CH_2$ | 4-phenyl-piperidino |
| H | 7-OMe | H | H | H | O | S | $CH_2CH_2$ | 4-phenyl-piperidino |
| H | 7-t-Bu | H | H | H | O | S | $CH_2$ | cis-3,5-dimethyl-piperidino |
| H | 7-t-Bu | H | H | H | S | O | $CH_2$ | cis-3,5-dimethyl-piperidino |
| 5-n-Bu | H | H | H | H | O | S | $CH_2$ | cis-3,5-dimethyl-piperidino |
| 5-OMe | H | H | H | H | S | O | $CH_2$ | cis-3,5-dimethyl-piperidino |
| 5-Cl | 6-Cl | H | H | H | O | S | $CH_2$ | 4-phenyl-piperidino |
| 6-t-Bu | H | H | H | H | O | S | $CH_2CH_2CH_2$ | cis-3,5-dimethyl-piperidino |
| H | 7-t-Bu | H | H | H | S | O | $CH_2CH_2CH_2$ | cis-3,5-dimethyl-piperidino |
| 6-1-Pr | H | H | H | H | O | S | $CH_2$ | cis-3,5-dimethyl-piperidino |
| H | 8-n-Bu | H | H | H | O | S | $CH_2$ | 4-phenyl-piperidino |

Formulations

The method of this invention can be conveniently carried out by formulating a compound of Formula I or its hydrochloride) in the conventional ways. In cases where the compound is a liquid at normal temperatures, it may be made into a solid formulation by absorption on a carrier. Possible formulation types include dusts granules, pellets, solutions, suspensions, emulsions, wettable powders, emulsifiable concentrates and the like. Many of the these may be applied directly. Sprayable formulations can be extended in suitable media and used at spray volumes of from a few liters to several hundred liters per hectare. High strength compositions are primarily used as intermediates for further formualtion. The formulations, broadly, contain about 0.1% to 99% by weight of active ingredient(s) and at least one of (a) about 0.1% to 2% surfactant(s) and (b) about 1% to 99.9% solid or liquid inter diluent(s). More specifically, they will contain these ingredients in the following approximate proportions:

| | Active Ingredient | Weight Percent* | |
|---|---|---|---|
| | | Diluent(s) | Surfactant(s) |
| Wettable Powders and water dispersible granules | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0° C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Water dispersible granules may be produced by agglomerating a fine powder composition (see, for example B. Cross and H. Scher, "Pesticide Formulations", ACS Symposium Series 371, American Chemical Society, Washington, DC, 1988, pp. 251-159). Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering, December 4, 1967, pp. 147ff. and "Perry's Chemical Engineer's Handbook", 5th Ed., McGraw-Hill, New York, 1973, pp. 8-57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138-140, 162-164, 166, 167 and 169-182;

H. Gysin and E. Knusli, U.S. Patent 2,891,855, June 23, 1959, Col. 3, line 66 through Col. 5, line 17 and Examples 1-4;

G. C. Klingman, "Weed Control as a Science", John Wiley and Sons, Inc., New York, 1961, pp. 81-96; and

J. D. Fryer and S. A. Evans, "Weed Control Hand- book", 5th Ed., Blackwell Scientific Publications, Oxford, 1968, pp. 101-103.

In the following examples, all parts are by weight unless otherwise indicated.

| Example 16 | |
|---|---|
| Low Strength Granule | |
| 1-[[2,3-dihydro-6-and-7-(1,1-dimethylethyl)-1,4-benzodioxan-2-yl]methyl]-3,5-dimethylpiperidine 0.1%<br>attapulgite granules<br>(U.S.S. 20 to 40 mesh) | <br><br>99.9% |

The active ingredient is dissolved in a solvent and the solution is sprayed upon dedusted granules in a double-cone blender. After spraying of the solution has been completed, the material is warmed to evaporate the solvent. The material is allowed to cool and then packaged.

| Example 17 | |
|---|---|
| Low Strength Granule | |
| 1-[[2,3-dihydro-6-and-7-(1,1-dimethylethyl)-1,4-benzodioxan-2-yl]methyl]-3,5-dimethylpiperidine 1%<br>N,N-dimethylformamide<br>attapulgite granules<br>(U.S.S. 20 to 40 sieve) | <br>9%<br>90% |

The active ingredient is dissolved in the solvent and the solution is sprayed upon dedusted granules in a double-cone blender. After spraying of the solution has been completed, the blender is allowed to run for a short period and then the granules are packaged.

Example 18

Emulsifiable Concentrate

| | |
|---|---|
| 1-[[2,3-dihydro-6-and-7-(1,1-dimethylethyl)-1,4-benzodioxan-2-yl]methyl]-3,5-dimethyl-piperidine | 35% |
| blend of calcium sulfonates and non-ionic surfactants | 6% |
| xylene | 59% |

The ingredients are combined and filtered to clarify the solution. The product can be used directly, extended with oils, or emulsified in water.

| Example 19 | |
|---|---|
| Dust | |
| 1-[[2,3-dihydro-6-and-7-(1,1-dimethylethyl)-1,4-benzodioxin-2-yl]methyl]-3,5-dimethylpiperidine 10% | 10% |
| attapulgite | 10% |
| Pyrophyllite | 80% |

The active ingredient is blended with attapulgite and then passed through a hammer-mill to produce particles substantially all below 200 microns. The ground concentrate is then blended with powdered pyrophyllite until homogeneous.

Utility

The compounds of this invention are useful as plant disease control agents. They are effective in controlling a broad spectrum of plant diseases, particularly foliar pathogens of ornamental, vegetable, field, cereal and fruit crops, such as *Puccinia recondita*, *Erysiphe graminis*, *Venturia inaequalis*, *Cercospora arachidicola*, *Botrytis cinerea*, *Cercosporidium personatum*, *Podosphaera leucotricha*, *Cercospora beticola*, and *Uncinula necatur*. They also control seed pathogens.

Disease control is ordinarily accomplished by applying an effective amount of the compound either pre- or post-infection to the portion of the plant to be protected, such as the roots, stems, foliage, fruit, seeds, tubers or bulbs, or to the media (soil or sand) in which the plants to be protected are growing. The compound may also be applied to the seed from which the plants to be protected are to be grown.

Rates of application for these compounds can be influenced by many factors of the environment and should be determined under actual use conditions. Foliage can normally be protected when treated at a rate of from less than 1 g/ha to 5000 g/ha of active ingredient. Plants growing in soil treated at a concentration from 0.1 to about 20 kg/ha can be protected from disease. Seed and seedlings can normally be protected when seed is treated at a rate of from 0.06 to about 3 grams per kilogram of seed.

The compounds of this invention can be mixed with fungicides, bactericides, acaricides, nematicides,

insecticides, or other biologically active compounds in order to achieve desired results with a minimum expenditure of time, effort and material. Amounts of these biologically active materials added for each part by weight of the composition of this invention may vary from 0.05 to 25 parts by weight. Suitable agents of this type are well known to those skilled in the art. Some are listed below:

Fungicides:

methyl 2-benzimidazolecarbamate (carbendazim)
tetramethylthiuram disulfide (thiuram)
n-dodecylguanidine acetate (dodine)
manganese ethylenebisdithiocarbamate (maneb)
1,4-dichloro-2,5-dimethoxybenzene (chloroneb)
methyl 1-(butylcarbamoyl)-2-benzimidazolecarbamate (benomyl)
2-cyano-N-ethylcarbamoyl-2-methoxyiminoacetamide (cymoxanil)
N-(trichloromethylthio)tetrahydrophthalimide (captan)
N-(trichloromethylthio)phthalimide (folpet)
dimethyl 4,4′-(o-phenylene)bis(3-thioallophanate)(thiophanate-methyl)
2-(thiazol-4-yl)-benzimidazole (thiabendazole)
aluminum tris (O-ethyl phosphonate)(phosethyl aluminum)
tetrachloroisophthalonitrile (chlorothalonil)
2,6-dichloro-4-nitroaniline (dichloran)
N-(2,6-dimethylphenyl)-N-(methoxyacetyl)alanine methyl ester (metalaxyl)
cis-N-[1,1,2,2-tetrachloroethyl)thio]cyclohex-4-ene-1,2-dicarbioximide (captafol)
3-(3,5-dichlorophenyl)-N-(1-methylethyl)-2,4-dioxo-1-imidazolidine carboxamide (iprodione)
3-(3,5-dichlorophenyl)-5-ethenyl-5-methyl-2,4-oxazolidinedione (vinclozolin)
kasugamycin
O-ethyl-S,S-diphenylphosphorodithioate(edifenphos)
4-(3-(4-(1,1-dimethylethyl)phenyl)-2-methyl)propyl-2,6-dimethylmorpholine (Fenpropimorph)
4-(3,4(1,1-dimethylethylphenyl)-2-methyl)propylpiperidine (Fenpropidine)
1-[[(bis(4-fluorophenyl)methylsilyl]methyl]-1H-1,2,4-triazole (flusilazole)
2-p-chlorophenyl-2-(1H-1,2,4-triazol-1-ylmethyl)hexanenitrile (myclobutanil)
(±)-1-[2-(2,4-dichlorophenyl)-4-propyl-1,3-dioxolan-2-ylmethyl]-1H-1,2,4-triazole (propiconazole)
N-propyl-N-[2-(2,4,6-trichlorophenoxy)ethyl]imidazole-1-carboxamide (prochloraz)
(RS)-2,4′-difluoro-α-(1H-1,2,4-triazol-1-ylmethyl)-benzhydryl alcohol (flutriafol)
1-(4-chlorophenoxy)-3,3-dimethyl-1-(1H-1,2,4-triazol-1-yl)butanone (triadimefon)
1-(4-chlorophenoxy)-3,3-dimethyl-1-(1H-1,2,4-triazol-1-yl)butan-2-ol (triadimenol)
(2RS,3RS)-1-(2,4-dichlorophenyl)-4,4-dimethyl-2-(1H,-1,2,4-triazol-1-yl)pentan-3-ol (diclobutrazol)

Bactericides:

tribasic copper sulfate
streptomycin sulfate.
oxytetracycline

Acaricides:

senecioic acid, ester with 2-sec-butyl-4,6-dinitrophenol (binapacryl)
6-methyl-1,3-dithiolo[2,3,B]quinonolin-2-one (oxythioquinox)
2,2,2-trichloro-1,1-bis(4-chlorophenyl)ethanol(dicofol)
bis(pentachloro-2,4-cyclopentadien-1-yl) (dienochlor)
tricyclohexyltin hydroxide (cyhexatin)
hexakis(2-methyl-2-phenylpropyl)distannoxane (fenbutin oxide)

Nematocides:

2-[diethoxyphosphinylimino]1,3-diethietane (fosthietan)
S-methyl 1-(dimethylcarbamoyl)-N-(methylcarbamoyloxy)thioformimidate(oxamyl)

S-methyl 1-carbamoyl-N-(methylcarbamoyloxy)thioformimidate
N-isopropylphosphoramidic acid, O-ethyl O′-[4-(methylthio)-m-tolyl]diester (fenamiphos).

Insecticides:

3-hydroxy-N-methylcrotonamide(dimethylphosphate)ester (monocrotophos)
methylcarbamic acid, ester with 2,3-dihydro-2,2-dimethyl-7-benzofuranol (carbofuran)
O-[2,4,5-trichloro-α-(chloromethyl)benzyl]phosphoric acid, O′,O′-dimethyl ester (tetrachlorvinphos)
2-mercaptosuccinic acid, diethyl ester, S-ester with thionophosphoric acid, dimethyl ester (malathion)
phosphorothioic acid, O,O-dimethyl, O-p-nitrophenyl ester (methyl parathion)
methylcarbamic acid, ester with α-naphthol (carbaryl)
methyl N-[[(methylamino)carbonyl]oxy]ethanimidothioate (methomyl)
N′-(4-chloro-o-tolyl)-N,N-dimethylformamidine (chlordimeform)
O,O-diethyl-O-(2-isopropyl-4-methyl-6-pyrimidyl)phosphorothioate (diazinon)
octachlorocamphene (toxaphene)
O-ethyl O-p-nitrophenyl phenylphosphonothioate (EPN)
cyano(3-phenoxyphenyl)-methyl-4-chloro-α-(1-methylethyl)benzeneacetate (fenvalerate)
(3-phenoxyphenyl)methyl(±)-cis,trans-3(2,2-dichloroethenyl)-2,2-dimethylcyclopropanecarboxylate (permethrin)
dimethyl N,N′-[thiobis(N-methylimino)carbonyloxy]]-bis[ethanimidothioate] (thiodicarb)
phosphorothiolothionic acid, O-ethyl-O-[4-(methylthio)phenyl]-S-n-propyl ester (sulprofos)
α-cyano-3-phenoxybenzyl 3-(2,2-dichlorovinyl)-2,2-dimethylcyclopropane carboxylate (cypermethrin)
cyano(3-phenoxyphenyl)methyl 4-(difluoromethoxy)-α-(methylethyl)benzeneacetate (flucythrinate)
O,O-diethyl-O-(3,5,6-trichloro-2-pyridyl)phosphorothioate (chlorphyrifos)
O,O-dimethyl-S-[[(4-oxo-1,2,3-benzotriazin-3-(4H)-yl)methyl]phosphorodithioate (azinphos-methyl)
5,6-dimethyl-2-dimethylamino-4-pyrimidinyl dimethyl carbamate (pirimicarb)
S-(N-formyl-N-methylcarbamoylmethyl)-O,O-dimethylphosphorodithioate (formothion)
S-2-(ethylthioethyl)-O,O-dimethyl phosphiorothioate (demeton-S-methyl)
α-cyano-3-phenoxybenzyl cis-3-(2,2-dibromovinyl)-2,2-dimethylcyclopropane carboxylate (deltamethrin)
cyano(3-phenoxyphenyl)methyl ester of N-(2-chloro-4-trifluoromethylphenyl)alanine (fluvalinate)

The fungicidal properties of the subject compounds were discovered in a number of greenhouse tests. The test procedures and results follow:

TABLE OF COMPOUNDS

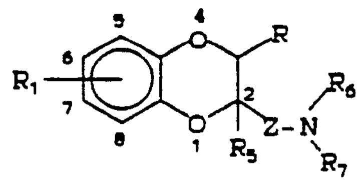

| CMPD | R1 | Z | R3 | R5 | NR6R7 |
|---|---|---|---|---|---|
| 1 | 6-t-butyl | $CH_2$ | H | H | piperidino |
| 2 | 7-t-butyl | $CH_2$ | H | H | morpholino |
| 3 | 6- and 7-t-butyl | $CH_2$ | H | H | cis-3,5-dimethyl-piperidino |
| 4 | 6- and 7-t-butyl | $CH_2$ | H | H | 4-methylpiperidino |
| 5 | 6- and 7-t-butyl | $CH_2CH_2$ | H | H | piperidino |
| 6 | 6- and 7-t-butyl | $CH_2$ | H | H | 4-phenylpiperidino |
| 7 | 6- and 7-t-butyl | $CH_2$ | H | H | 3,3-dimethylpiperidino |
| 8 | 6- and 7-t-butyl | $CH_2CH_2$ | H | H | trans-3,5-dimethyl-piperidino |
| 9 | 6- and 7-t-butyl | $CH_2CH_2$ | H | H | cis-3,5-dimethyl-piperidino |
| 10 | 6- and 7-t-butyl | $CH_2$ | H | H | trans-3,5-dimethyl-piperidino |
| 11 | 6- and 7-t-butyl | $CH_2$ | H | H | 2,6-dimethylmorpholino |
| 12 | 6-t-butyl | $CH_2$ | H | H | cis-3,5-dimethyl-piperidino |

| CMPD | $R_1$ | Z | $R_3$ | $R_5$ | $NR_6R_7$ |
|---|---|---|---|---|---|
| 13 | 6-t-butyl | $CH_2$ | H | H | trans-3,5-dimethyl-piperidino |
| 14 | 6- and 7-t-butyl | $CH_2$ | H | H | cis-3,5-dimethyl-piperidino HCl Salt (mp 205-217°) |
| 15 | 7-t-butyl | $CH_2$ | H | H | cis-3,5-dimethyl-piperidino |
| 16 | 6- and 7-t-butyl | $CH_2$ | H | H | hexamethyleneimino |
| 17 | 6- and 7-t-butyl | $CH_2$ | H | H | N,N-di-n-butylamino |
| 18 | 6- and 7-t-butyl | $CH_2$ | H | H | heptamethyleneimino |
| 19 | 6- and 7-t-butyl | $CH_2CH_2$ | H | H | cis-3,5-dimethyl-piperidino HCl Salt (mp 207-209°) |
| 20 | 6- and 7-t-butyl (Isomer A) | $CH_2$ | $CH_3$ | $CH_3$ | piperidino |
| 21 | 6- and 7-t-butyl (Isomer B) | $CH_2$ | $CH_3$ | H | piperidino |
| 22 | 6- and 7-t-butyl | $CH_2$ | H | H | piperidino |
| 23 | 6- and 7-t-butyl | $CH_2CH_2$ | H | H | hexamethyleneimino |
| 24 | 6- and 7-t-butyl (Isomer A) | $CH(CH_3)CH_2$ | H | H | piperidino |
| 25 | 6- and 7-t-butyl | $CH_2CH_2$ | $CH_3$ | H | piperidino |
| 26 | 6- and 7-t-butyl | $CH_2CH_2$ | H | $CH_3$ | piperidino |
| 27 | 6- and 7-t-butyl | $CH_2CH_2$ | $CH_3$ | H | $N(n\text{-}Bu)_2$ |
| 28 | 6- and 7-t-butyl | $CH_2CH_2$ | $CH_3$ | H | cis-3,5-dimethyl-piperidino |
| 29 | 6- and 7-t-butyl (Isomer B) | $CH(CH_3)CH_2$ | H | H | piperidino |
| 30 | 6- and 7-t-butyl (Isomer A) | $CH(CH_3)CH_2$ | H | H | cis-3,5-dimethyl-piperidino |
| 31 | 6- and 7-t-butyl (Isomer B) | $CH(CH_3)CH_2$ | H | H | cis-3,5-dimethyl-piperidino |
| 32 | 6- and 7-t-butyl | $CH_2CH_2CH_2$ | H | H | piperidino |

| CMPD | $R_1$ | Z | $R_3$ | $R_5$ | $NR_6R_7$ |
|---|---|---|---|---|---|
| 33 | 6- and 7-t-butyl | $CH_2$ | H | H | 3-methylpiperidino |
| 34 | 6- and 7-t-butyl | $CH_2$ | H | H | cis-3,5-dimethyl-piperidino |
| 35 | 6- and 7-t-butyl | $CH_2CH_2$ | H | $CH_3$ | cis-3,5-dimethyl-piperidino |
| 36 | 6- and 7-t-butyl | $CH_2CH_2$ | H | $CH_3$ | 4-phenylpiperidino |
| 37 | 6- and 7-t-butyl | $CH_2$ | H | H | $NEt_2$ |
| 38 | H | $CH_2$ | H | H | 4-phenylpiperidino |
| 39 | H | $CH_2$ | H | H | cis-3,5-dimethyl-piperidino |
| 40 | 6- and 7-t-butyl | $CH_2CH_2$ | H | $CH_3$ | cis-2,6-dimethyl-morpholino |
| 41 | 6- and 7-t-butyl (Isomer A) | $CHCH_3$ | H | H | cis-3,5-dimethyl-piperidino |
| 42 | 6- and 7-t-butyl | $CHCH_3$ | H | H | cis-3,5-dimethyl-piperidino |
| 43 | 6- and 7-t-butyl (Isomer A) | $CH_2$ | $CH_3$ | H | cis-3,5-dimethyl-piperidino |
| 44 | 6 and 7-t-butyl (Isomer B) | $CH_2$ | $CH_3$ | H | cis-3,5-dimethyl-piperidino |
| 45 | 6- and 7-t-butyl | $CH_2CH_2$ | H | H | cis-2,6-dimethyl-morpholino |
| 46 | 6- and 7-t-butyl | $CH_2CH_2CH_2$ | H | H | cis-3,5-dimethyl-piperidino |
| 47 | 5-$OCH_3$ | $CH_2$ | H | H | piperidino |
| 48 | 6- and 7-t-butyl | $CH_2$ | H | H | cis-2,6-dimethyl-morpholino HCl Salt (mp 232-234°) |
| 49 | 6- and 7-$C(CH_3)_2CH_2C(CH_3)_3$ | $CH_2$ | H | H | cis-3,5-dimethyl-piperidino |
| 50 | 6- and 7-t-butyl | $CH_2CH_2$ | H | H | heptamethyleneimino |
| 51 | 6- and 7-t-butyl | $CH_2CH_2$ | H | H | 4-phenylpiperidino |
| 52 | 6- and 7-t-butyl | $CH_2CH_2$ | H | H | trans-2,6-dimethyl-morpholino |

| CMPD | $R_1$ | Z | $R_3$ | $R_5$ | $NR_6R_7$ |
|---|---|---|---|---|---|
| 53 | 6- and 7-$C_6H_5$ | $CH_2CH_2$ | H | H | piperidino |
| 54 | 6- and 7-$C(CH_3)_2CH_2C(CH_3)_3$ | $CH_2$ | H | H | piperidino |
| 55 | 6- and 7-$C(CH_3)_2CH_2C(CH_3)_3$ | $CH_2$ | H | H | piperidino |

## NMR SPECTRAL DATA (in $CDCl_3$)*

| CMPD | | |
|---|---|---|
| 1 | PPM: | 1.25-1.70 (m, 15H), 2.40-2.70 (m, 6H), 3.92-4.30 (m, 1H), 4.25-4.38 (m, 2H), 6.78-6.90 (m, 3H) |
| 2 | PPM: | 1.28 (s, 9H), 2.50-2.75 (m, 6H), 3.74 (t, 4H), 3.98 (q, 1H), 4.25-4.38 (m, 2H), 6.80-6.95 (m, 3H) |
| 3 | PPM: | 0.55 (broad q, 1H), 0.85 (broad d, 6H), 1.27 (s, 9H), 1.50-1.80 (m, 5H), 2.48-2.72 (m, 2H), 2.77-2.95 (m, 2H), 3.88-4.03 (m, 1H), 4.25-4.38 (m, 2H), 6.78-6.93 (m, 3H) |
| 4 | PPM: | 0.93 (d, 3H), 1.18-1.45 (m, 11H), 1.55-1.70 (m, 2H), 1.95-2.20 (m, 2H), 2.47-2.70 (m, 2H), 2.82-3.05 (m, 2H), 3.87-4.03 (m, 1H), 4.22-4.35 (m, 2H), 6.78-6.95 (m, 3H) |
| 5 | PPM: | 1.28 (s, 9H), 1.40-2.0 (m, 8H), 2.30-2.63 (m, 6H), 3.80-3.97 (m, 1H), 4.10-4.30 (m, 2H), 6.78-6.90 (m, 3H) |

*Chemical shifts are expressed in parts per million downfield from tetramethylsilane.

| CMPD | | |
|---|---|---|
| 6 | PPM: | 1.29 (s, 9H), 1.75-2.00 (m, 4H), 2.15-2.40 (m, 2H), 2.45-2.82 (m, 3H), 3.00-3.25 (m, 2H), 3.93-4.13 (m, 1H), 4.30-4.45 (m, 2H), 6.80-7.0 (m, 3H), 7.20-7.40 (m, 5H) |
| 7 | PPM: | 0.99 (s, 9H), 1.22-1.40 (m, 11H), 1.58-1.70 (m, 2H), 2.18 (q, 2H), 2.30-2.70 (m, 4H), 3.95-4.10 (m, 1H), 4.22-4.43 (m, 2H), 6.80-6.95 (m, 3H) |
| 8 | PPM: | 0.96 (d, 6H), 1.28 (broad s, 2H), 1.68-2.18 (m, 6H), 2.25-2.60 (m, 4H), 3.85-4.0 (m, 1H), 4.15-4.33 (m, 2H), 6.78-6.92 (m, 3H) |
| 9 | PPM: | 0.53 (broad q, 1H), 0.86 (d, 6H), 1.28 (s, 9H), 1.35-2.0 (m, 7H), 2.40-2.70 (m, 2H), 2.88 (broad d, 2H), 3.85-3.96 (m, 1H), 4.12-4.30 (m, 2H), 6.75-6.93 (m, 3H) |
| 10 | PPM: | 0.96 (broad d, 6H), 1.28 (broad s, 11H), 1.80-2.30 (m, 4H), 2.38-2.60 (m, 4H), 3.90-4.10 (m, 1H), 4.25-4.40 (m, 2H), 6.75-6.95 (m, 3H) |
| 11 | PPM: | 1.14-1.20 (m, 6H), 1.28 (s, 9H), 1.82-2.0 (m, 2H), 2.50-2.88 (m, 4H), 3.63-3.80 (m, 2H), 3.90-4.48 (m, 1H), 4.30 (broad d, 2H), 6.78-6.95 (m, 3H) |

| CMPD | | |
|---|---|---|
| 12 | PPM: | 0.53 (broad 1, 1H), 0.86 (broad d, 6H), 1.28 (s, 9H), 1.55-1.80 (m, 5H), 2.50-2.70 (m, 2H), 2.80-2.95 (m, 2H), 3.98 (dd, 1H), 4.25-4.35 (m, 2H), 6.80-6.93 (m, 3H) |
| 13 | PPM: | 0.96 (broad d, 6H), 1.28 (broad s, 11H), 1.68-2.30 (m, 4H), 2.40-2.68 (m, 4H), 3.95-4.08 (m, 1H), 4.20-4.40 (m, 2H), 6.75-6.95 (m, 3H) |
| 14 | PPM: | 0.52 (broad q, 1H), 0.86 (d, 6H), 1.29 (s, 9H), 1.50-1.80 (m, 5H), 2.45-2.73 (m, 2H), 2.78-2.95 (m, 2H), 3.93 (dd, 1H), 4.22-4.38 (m, 2H), 6.75-6.95 (m, 3H) |
| 15 | PPM: | 1.29 (s, 9H), 1.62 (broad s, 8H), 2.63-2.95 (m, 6H), 3.90-4.05 (m, 1H), 4.17-4.40 (m, 2H), 6.78-6.95 (m, 3H) |
| 16 | PPM: | 0.92 (t, 6H), 1.21-1.52 (m, 17H), 2.37-2.80 (m, 6H), 3.90-4.03 (m, 1H), 4.12-4.40 (m, 2H), 6.75-6.95 (m, 3H) |
| 17 | PPM: | 1.27 (s, 9H), 1.58 (broad s, 10H), 2.55-1.90 (m, 6H), 3.92-4.07 (m, 1H), 4.10-4.29 (m, 1H), 4.41 (d, 1H), 6.75-6.93 (m, 3H) |
| 18 | PPM: | 1.23-1.70 (m, 18H), 2.35-2.36 (m, 6H), 3.87-4.0 (m, 1H), 4.24-4.45 (m, 1H), 6.75-6.95 (m, 3H) |

| CMPD | | |
|---|---|---|
| 19 | PPM: | 1.20-1.65 (m, 18H), 2.25-2.60 (m, 6H), 3.83-3.95 (m, 1H), 4.10-4.40 (m, 1H), 6.75-6.90 (m, 3H) |
| 20 | PPM: | 1.20-1.70 (m, 15H), 2.35-2.72 (m, 6H), 3.90-4.05 (m, 1H), 4.23-4.38 (m, 2H), 6.78-6.95 (m, 3H) |
| 21 | PPM: | 1.30 (s, 9H), 1.50-2.0 (m, 10H), 2.55-2.80 (m, 6H), 3.85-4.0 (m, 1H), 4.15-4.33 (m, 2H), 6.75-6.95 (m, 3H) |
| 22 | PPM: | 1.01 (dd, 3H), 1.20-1.70 (m, 16H), 2.05-2.50 (m, 6H), 3.95-4.15 (m, 2H), 4.30-4.40 (m, 1H), 6.75-6.95 (m, 3H) |
| 23 | PPM: | 1.20-1.95 (m, 20H), 2.30-2.75 (m, 6H), 3.72-4.40 (m's, 2H) |
| 24 | PPM: | 1.20-1.70 (m, 18H), 1.75-1.95 (m, 2H), 2.25-2.60 (m, 6H), 3.82-4.0 (m, 2H), 6.70-6.95 (m, 3H) |
| 25 | PPM: | 0.93 (t, 6H), 1.18-1.55 (m, 20H), 1.56-2.0 (m, 2H), 2.27-2.80 (m, 6H), 3.77-4.40 (m's, 2H), 6.73-6.95 (m, 3H) |
| 26 | PPM: | 0.52 (broad q, 1H), 0.80-1.00 (m, 6H), 1.20-1.95 (m, 19H), 0.40-2.75 (m, 2H), 2.80-2.95 (m, 2H), 3.70-4.40 (m's, 2H), 6.73-6.95 (m, 3H) |

| CMPD | | |
|---|---|---|
| 27 | PPM: | 1.05 (broad d, 6H), 1.20-1.70 (m, 19H), 2.05-2.56 (m, 6H), 4.0-4.30 (m, 3H), 6.75-6.95 (m, 3H) |
| 28 | PPM: | 0.52 (broad q, 1H), 0.85 (d, 6H), 0.95-1.80 (m's, 18H), 0.92-2.53 (m, 3H), 2.80 (broad t, 1H), 3.95-4.40 (m, 3H), 6.75-6.95 (m, 3H) |
| 29 | PPM: | 0.52 (broad q, 1H0, 0.80-0.93 (m, 6H), 0.95-1.78 (m, 19H), 2.10-2.90 (m's, 4H), 4.0-4.45 (m, 3H), 6.75-6.95 (m, 3H) |
| 30 | PPM: | 1.22-1.90 (m, 19H), 2.25-2.50 (m, 6H), 3.78-4.30 (m's, 3H), 6.75-6.95 (m, 3H) |
| 31 | PPM: | 0.80 (d, 6H), 1.27 (s, 9H), 0.50-1.85 (m, 9H), 1.87-2.12 (m, 1H), 2.43-3.0 (m, 4H), 3.90-4.05 (m, 1H), 4.22-4.37 (m, 2H), 6.75-6.95 (m, 3H) |
| 32 | PPM | 0.40-1.00 (m, 6H), 1.22-2.05 (m, 8H), 2.41-2.68 (m, 2H), 2.78-2.95 (m, 2H), 3.89-4.04 (m, 1H), 4.18-4.39 (m, 2H), 6.88-7.61 (m, 8H) |
| 33 | PPM: | 0.52 (broad q, 1H), 0.85 (broad d, 6H), 1.25-2.0 (m's, 19H), 2.43-2.55 (m, 2H), 2.78-2.90 (m, 2H), 3.83-4.0 (m, 2H), 6.75-6.95 (m, 3H) |

| CMPD | | |
|---|---|---|
| 34 | PPM: | 1.20-1.40 (m, 12H), 1.70-2.15 (m, 8H), 2.40-2.63 (m, 3H), 3.0-3.13 (m, 2H), 3.85-4.05 (m, 2H), 6.75-6.95 (m, 3H), 7.15-7.45 (m, 5H) |
| 35 | PPM: | 1.05 (t, 6H), 1.27 (s, 9H), 2.50-2.80 (m, 6H), 3.90-4.38 (m's, 3H), 6.75-6.95 (m, 3H) |
| 36 | PPM: | 1.53-2.80 (m's, 9H), 3.0-3.22 (m, 2H), 4.02 (dd, 1H), 4.30-4.45 (m, 2H), 6.80-6.95 (m, 3H), 7.15-7.40 (m, 5H) |
| 37 | PPM: | 0.53 (broad q, 1H), 0.87 (broad d, 6H), 1.53-1.83 (m, 5H), 2.50-2.73 (m, 2H), 2.79-2.97 (q, 2H), 3.97 (dd, 1H), 4.28-4.40 (m, 2H), 6.70-6.95 (m, 3H) |
| 38 | PPM: | 1.10-1.40 (m, 18H), 1.60-1.95 (m, 4H), 2.43-2.55 (m, 2H), 2.74 (braod d, 2H), 3.55-3.75 (m, 2H), 3.92 (broad q, 2H), 6.75-6.95 (m, 3H) |
| 39 | PPM: | 0.51 (broad q, 1H), 0.83 (d, 6H), 1.07-2.80 (m's, 20H), 4.0-4.50 (m, 3H), 6.75-7.05 (m, 3H) |
| 40 | PPM: | 0.51 (broad q, 1H), 0.75-3.0 (m's, 26H), 4.0-4.40 (m, 3H), 6.75-7.05 (m, 3H) |

| CMPD | | |
|---|---|---|
| 41 | PPM: | 0.51 (broad q, 1H), 0.80-1.80 (m's, 23H), 2.62 (d, 2H), 2.88 (broad t, 2H), 4.0-4.40 (m, 2H), 6.70-6.95 (m, 3H) |
| 42 | PPM: | 0.52 (braod q, 1H), 0.85 (d, 6H), 1.20-1.40 (m, 12H), 1.50-1.80 (m, 5H), 2.43-2.65 (m, 2H), 3.88 (dd, 2H), 4.25-4.43 (m, 2H), 6.75-6.95 (m, 3H) |
| 43 | PPM: | 1.13-1.35, 1.60-1.97, 2.42-2.63 (m's, 21H), 2.75 (d, 2H), 3.57-4.30 (m's, 5H), 6.75-6.95 (m, 3H) |
| 44 | PPM: | 0.51 (broad q, 1H), 0.85 (d, 6H), 1.20-1.90 (m's, 18H), 2.37 (t, 2H), 2.83 (d, 2H), 3.80-4.30 (m's, 3H), 6.75-6.95 (m, 3H) |
| 45 | PPM: | 1.35-1.70 (m, 6H), 2.35-2.80 (m, 6H), 3.87 (broad s, 3H), 3.95-4.60 (m's, 3H), 6.43-6.90 (m's, 3H) |
| 46 | PPM: | 0.40-0.95 (m, 15H), 1.20-1.48 (m, 7H), 1.50-1.82 (m, 7H), 2.48-2.70 (m, 2H), 2.75-2.98 (m, 2H), 3.88-4.05 (m, 1H), 4.20-4.45 (m, 2H), 6.73-7.00 (m, 3H) |
| 47 | PPM: | 1.29 (s, 9H), 1.50-1.95 (m, 12H), 2.50-2.70 (m, 6H), 3.83-3.97 (m, 1H), 4.20-4.35 (m, 2H), 6.75-6.95 (m, 3H) |

| CMPD | | |
|---|---|---|
| 48 | PPM: | 1.29 (s, 9H), 1.70-2.20 (m, 8H), 2.40-2.70 (m, 3H), 3.09 (broad d, 2H), 3.85-4.00 (m, 1H), 4.15-4.32 (m, 2H), 6.75-6.95 (m, 3H), 7.18-7.39 (m, 5H) |
| 49 | PPM: | 1.20-2.55 (m's, 23H), 3.85-4.35 (m's, 5H), 6.75-6.95 (m, 3H) |
| 53 | PPM: | 1.37-2.04 (m, 8H), 2.29-2.70 (m, 6H), 3.89-4.02 (m, 1H), 4.20-4.40 (m, 2H), 6.89-7.60 (m, 8H) |
| 54 | PPM: | 0.73 (s, 9H), 1.15-1.83 (m, 16H), 2.43-2.72 (m, 3H), 3.38-3.49 (m, 1H), 3.85-4.03 (m, 1H), 4.20-4.43 (m, 2H), 6.72-6.95 (m, 3H) |
| 55 | PPM: | 0.72 (s, 9H), 1.30 (s, 6H), 1.38-2.00 (m, 10H), 2.30-2.64 (m, 5H), 3.81-4.00 (m, 1H), 4.08-4.28 (m, 2H), 6.70-6.90 (m, 3H) |

Test A

The test compounds were dissolved in acetone in an amount equal to 6% of the final volume and then suspended at a concentration of from 1000 to 2 ppm in purified water containing 250ppm of the surfactant TREM 014 (polyhydric alcohol ester). This suspension was sprayed to the point of run-off on wheat seedlings. The following day plants were inoculated with a spore suspension of Puccinia recondita, the causal agent of wheat leaf rust, and incubated in a saturated humidity chamber at 20° C for 24 hours and then in a growth chamber at 20° C for 7 days, when disease ratings were made.

Test B

The test compounds were dissolved in acetone in an amount equal to 6% of the final volume and then suspended at a concentration of from 1000 to 2 ppm in purified water containing 250ppm of the surfactant TREM 014 (polyhydric alcohol esters). This suspension was sprayed to the point of run-off on broad bean or cucumber seedlings. The following day plants were inoculated with a spore suspension of Botrytis cinerea, the causal agent of grey mold, and incubated in a saturated humidity chamber at 20° C for 48 hours and then in a growth chamber at 20° C for 4 days, when disease ratings were made.

Test C

The test compounds were dissolved in acetone in an amount equal to 6% of the final volume and then suspended at a concentration of from 1000 to 2 ppm in purified water containing 250ppm of the surfactant TREM 014 (polyhydric alcohol esters). This suspension was sprayed to the point of run-off on wheat seedlings. The following day plants were inoculated with a spore dust of Erysiphe graminis f. sp. tritici, the causal agent of wheat powdery mildew, and incubated in a growth chamber at 20° C for 7 days, when disease ratings were made.

Test D

The test compounds were dissolved in acetone in an amount equal to 6% of the final volume and then suspended at a concentration of from 1000 to 2 ppm in purified water containing 250ppm of the surfactant TREM 014 (polyhydric alcohol esters). This suspension was sprayed to the point of run-off on peanut seedlings. The following day plants were inoculated with a spore suspension of Cercosporidium personatum, the causal agent of peanut late leaf spot, and incubated in a saturated humidity chamber at 22° C for 24 hours and then in a high humidity chamber at 27° C for 7 days, and then in a growth chamber at 29° C for 4 days, when disease ratings were made.

Test E

The test compounds were dissolved in acetone in an amount equal to 6% of the final volume and then suspended at a concentration of from 1000 to 2 ppm in purified water containing 250ppm of the surfactant TREM 014 (polyhydric alcohol esters). This suspension was sprayed to the point of run-off on tomato seedlings. The following day plants were inoculated with a spore suspension of Phytophthora infestans, the causal agent of tomato late blight, and incubated in a saturated humidity chamber at 20° C for 24 hours and then in a growth chamber at 20° C for 4 days, when disease ratings were made.

Test F

The test compounds were dissolved in acetone in an amount equal to 6% of the final volume and then suspended at a concentration of from 1000 to 2 ppm in purified water containing 250ppm of the surfactant TREM 014 (polyhydric alcohol esters). This suspension was sprayed to the point of run-off on apple seedlings. The following day plants were inoculated with a spore suspension of Venturia inaequalis, the causal agent of apple scab, and incubated in a saturated humidity chamber at 20° C for 24 hours and then in a growth chamber at 22° C for 11 days, when disease ratings were made.

Test G

The test compounds were dissolved in acetone in an amount equal to 6% of the final volume and then suspended at a concentration of from 1000 to 2 ppm in purified water containing 250ppm of the surfactant TREM 014 (polyhydric alcohol esters). This suspension was sprayed to the point of run-off on apple seedlings. The following day plants were inoculated with a spore suspension of Podosphaera leucotricha, the causal agent of apple powdery mildew, and incubated in a greenhouse for 7 days, when disease ratings were made.

Test H

The test compounds were dissolved in acetone in an amount equal to 6% of the final volume and then suspended at a concentration of from 1000 to 2 ppm in purified water containing 250ppm of the surfactant TREM 014 (polyhydric alcohol esters). This suspension was sprayed to the point of run-off on cucumber seedlings. The following day plants were inoculated with a spore suspension of Sphaerotheca fuliginea, the causal agent of cucumber powdery mildew, and incubated in a greenhouse for seven days when disease

ratings were made.

Test I

The test compounds were dissolved in acetone in an amount equal to 6% of the final volume and then suspended at a concentration of from 1000 to 2 ppm in purified water containing 250ppm of the surfactant TREM 014 (polyhydric alcohol esters). This suspension was sprayed to the point of run-off on sugarbeet seedlings. The following day plants were inoculated with a spore suspension of Cercospora beticola, the causal agent of sugarbeet leaf spot, and incubated in a saturated humidity chamber at 22° C for 24 hours and then in a high humidity growth chamber at 22° C for 7 days, when disease ratings were made.

Test J

The test compounds were dissolved in acetone in an amount equal to 6% of the final volume and then suspended at a concentration of from 1000 to 2 ppm in purified water containing 250ppm of the surfactant TREM 014 (polyhydric alcohol esters). This suspension was sprayed to the point of run-off on grape seedlings. The following day plants were inoculated with a spore suspension of Plasmopara viticola, the causal agent of grape downy mildew, and incubated in a dew chamber at 20° C for 24 hours and then in a growth chamber at 20° C for 7 days, when disease ratings were made.

Results for Test A-J are given in Tables I and II. In these tables, a rating of 100 indicates 100% disease control and a rating of 0 indicates no disease control relative to un-treated controls. A -entry indicates that no test was performed with the specific compound at that specific rate.

## Table I

| CMPD | RATE PPM | A | B | C | D | E | F |
|---|---|---|---|---|---|---|---|
| 1 | 200 | 100 | 42 | 100 | 69 | 0 | 0 |
| 2 | 200 | 0 | 0 | 0 | 63 | 0 | 0 |
| 3 | 200 | 100 | 89 | 99 | 98 | 0 | 64 |
| 4 | 200 | 0 | 78 | 91 | 76 | 0 | 33 |
| 5 | 200 | 85 | 99 | 85 | 97 | 0 | 0 |
| 6 | 200 | 45 | 71 | 91 | 91 | 0 | 100 |
| 7 | 200 | - | 0 | 0 | 16 | 0 | 15 |
| 8 | 200 | 98 | 93 | 97 | 99 | 25 | 96 |
| 9 | 200 | 100 | 88 | 95 | 99 | 0 | 96 |
| 10 | 200 | 99 | 93 | 98 | 88 | 26 | 73 |
| 11 | 200 | 63 | 0 | 77 | 12 | 0 | 73 |
| 12 | 200 | 99 | 97 | 99 | 95 | 26 | 73 |
| 13 | 200 | 99 | 10 | 99 | 52 | 26 | 73 |
| 14 | 200 | 100 | 0 | 99 | 100 | 0 | 92 |
| 15 | 200 | 79 | 0 | 95 | 0 | 0 | 100 |
| 16 | 200 | 99 | 0 | 95 | 0 | 0 | 88 |
| 17 | 200 | 0 | 0 | 76 | 0 | 0 | 39 |
| 18 | 200 | 89 | 0 | 77 | 26 | 0 | 76 |
| 19 | 200 | 100 | 47 | 99 | 96 | 77 | 100 |
| 20 | 200 | 82 | 97 | 100 | 11 | - | 100 |
| 21 | 200 | 99 | 0 | 100 | 78 | 0 | 100 |
| 22 | 200 | 99 | 0 | 99 | 69 | 0 | 88 |
| 23 | 200 | 100 | 0 | 99 | 94 | 47 | 95 |
| 24 | 200 | 96 | 48 | 99 | 85 | 0 | 100 |
| 25 | 200 | 18 | 0 | 37 | 0 | 47 | 98 |
| 26 | 200 | 100 | 81 | 99 | 89 | - | 93 |
| 27 | 200 | 17 | 0 | 86 | 29 | 45 | 80 |
| 28 | 200 | 33 | 0 | 77 | 29 | 92 | 100 |
| 29 | 200 | 52 | 1 | 84 | 29 | - | 67 |

| | | | | | | | |
|---|---|---|---|---|---|---|---|
| 30 | 200 | 83 | 0 | 98 | 82 | 46 | 92 |
| 31 | 200 | 32 | 0 | 95 | 0 | 0 | 82 |
| 32 | 200 | 99 | 0 | 95 | 34 | 46. | 92 |
| 33 | 200 | 100 | 0 | 98 | 82 | 0 | 82 |
| 34 | 200 | - | 0 | 61 | 27 | 63 | 92 |
| 35 | 200 | 100 | 0 | 95 | 68 | 26 | 98 |
| 36 | 200 | 100 | 0 | 57 | 63 | 63 | 82 |
| 37 | 200 | 0 | 0 | 91 | 63 | 0 | 82 |
| 38 | 200 | 48 | 0 | 94 | 64 | 0 | 84 |
| 39 | 200 | - | 0 | 0 | 0 | 0 | 23 |
| 40 | 200 | 0 | 0 | 97 | 26 | 0 | 79 |
| 41 | 200 | 58 | 3 | 95 | 0 | - | 26 |
| 42 | 200 | 58 | 31 | 89 | 0 | - | 96 |
| 43 | 200 | 47 | 1 | 0 | 0 | - | 100 |
| 44 | 200 | 100 | 99 | 100 | 13 | - | 100 |
| 45 | 200 | 88 | 0 | 98 | 88 | 0 | 78 |
| 46 | 200 | 61 | 0 | 86 | 0 | 46 | 91 |
| 47 | 200 | - | 0 | 0 | 40 | 0 | 0 |
| 48 | 200 | - | 0 | 0 | 29 | 0 | 41 |
| 49 | 200 | 23 | 0 | 0 | 0 | 0 | 100 |

Table II

| CMPD | RATE PPM | A | B | C | D | F | G | H | I | J |
|---|---|---|---|---|---|---|---|---|---|---|
| 3 | 500 | - | - | - | - | - | 96 | 78 | 100 | - |
| | 200 | 100 | 94 | 99 | 96 | 98 | 93 | 34 | 79 | 21 |
| | 40 | 99 | 21 | 97 | 60 | 64 | 47 | 0 | - | - |
| | 10 | 43 | - | 83 | - | - | - | - | - | - |
| | 2 | 17 | - | 43 | - | - | - | - | - | - |
| 9 | 1000 | - | - | - | - | - | 98 | 99 | 100 | - |
| | 200 | 100 | 80 | 96 | 92 | 10 | 93 | 87 | 86 | - |
| | 40 | 59 | 42 | 96 | 13 | 87 | 43 | 37 | - | - |
| | 10 | 0 | - | 58 | 13 | 26 | 20 | 0 | - | - |
| | 2 | - | - | 20 | - | - | - | - | - | - |
| 8 | 200 | 98 | 80 | 95 | 93 | 100 | - | - | - | - |
| | 40 | 76 | 48 | 95 | 27 | 65 | - | - | - | - |
| | 10 | 12 | - | 74 | 27 | 33 | - | - | - | - |
| | 2 | - | - | 87 | - | - | - | - | - | - |
| 12 | 1000 | - | - | - | - | - | 98 | 97 | 100 | - |
| | 200 | 99 | 72 | 93 | 77 | 96 | 83 | 58 | 86 | 90 |
| | 40 | 100 | 29 | 81 | 0 | - | 10 | 9 | - | - |
| | 10 | 18 | - | 13 | - | - | 0 | 9 | - | - |
| | 2 | 0 | - | 0 | - | - | - | - | - | - |

## Claims

1. A method for controlling fungal diseases in plants comprising applying to the locus of the plant a compound of Formula I

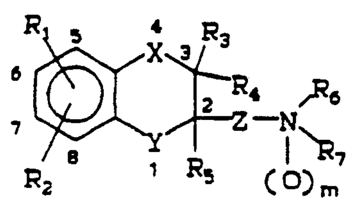

I

wherein

$R_1$ and $R_2$ are independently H, halogen, $C_1$-$C_8$ alkyl, $C_2$-$C_8$ alkenyl, $C_2$-$C_8$ alkynyl, $C_1$-$C_8$ alkoxy, $C_1$-$C_5$ alkyl substituted with 1-3 F, Cl or Br or $C_2$-$C_4$ alkoxyalkyl, $Si(CH_3)_3$, thioalkyl, or phenyl substituted with one or two of the following: halogen, $C_1$-$C_4$ alkyl or $C_1$-$C_4$ alkoxy.

$R_3$, $R_4$ and $R_5$ are independently H, $CH_3$, $CF_3$ or $CH_2CH_3$;

$R_6$ and $R_7$ are independently $C_1$-$C_8$ alkyl; or $R_6$ and $R_7$ can be taken together as piperidino, morpholino, pyrrolidino, hexamethyleneimino, heptamethyleneimino, wherein these groups are optionally substituted by 1 or 2 of the following: $C_1$-$C_4$ alkyl groups or phenyl rings substituted by hydrogen or 1 or 2 halogen, $C_1$-$C_4$ alkyl, or $C_1$ or $C_4$ alkoxy groups.

X is O or S;

Y is O or S;

Z is $C_1$-$C_4$ unbranched alkylene optionally substituted by 1-2 $CH_3$, $CF_3$ or $CH_2CH_3$ groups;

m is O or 1; and

their agriculturally suitable salts.

2. The method of Claim 1 wherein
$R_1$ and $R_2$ are located at the 6 and 7 positions of the benzene ring; and
$R_3$, $R_4$ and $R_5$ are independently H or $CH_3$.

3. The method of Claim 2 wherein
$R_1$ is H, $R_2$ is $C_1$-$C_4$ alkyl, $C_1$-$C_4$ alkoxy, $C_1$-$C_4$ alkoxyalkyl or halogen; and
$R_6$ and $R_7$ are taken together as piperidino, morpholino or pyrrolidino optionally substituted by phenyl or 1-2 $C_1$-$C_2$ alkyl groups.

4. The method of Claim 3 wherein
Z is $C_1$-$C_3$ unbranched alkylene optionally substituted by a $CH_3$ group.

5. The method of Claim 4 wherein
$R_6$ and $R_7$ are taken together as piperidino, morpholino, pyrrolidino, 2,6-dimethylmorpholino, 3,5-dimethylpiperidino and 4-phenylpiperidino;
$R_2$ is $C_2$-$C_4$ alkyl or $C_2$-$C_4$ alkoxyalkyl;
X and Y are O.

6. The method of Claim 1 wherein the compound is:
1-[2-[6-and-7-(1,1-dimethylethyl)-2,3-dihydro-1,4-benzodioxan-2-yl]ethyl]-trans-3,5-dimethylpiperidine.

7. The method of Claim 1 wherein the compound is:
1-[2-[6-and-7-(1,1-dimethylethyl)-2,3-dihydro-1,4-benzodioxan-2-yl-ethyl]-cis-3,5-dimethylpiperidine.

8. The method of Claim 1 wherein the compound is:
1-[[2,3-dihydro-6-and-7-(1,1-dimethylethyl)-1,4-benzodioxan-2-yl]methyl]-cis-3,5-dimethylpiperidine.

9. The method of Claim 1 wherein the compound is:
1-[[6-(1,1-dimethylethyl)-2,3-dihydro-1,4-benzodioxan-2-yl]methyl]piperidine.

10. The method of Claim 1 wherein the compound is:
1-[2-[6-and-7-(1,1-dimethylethyl)-2,3-dihydro-1,4-benzodioxan-2-yl]ethyl]-cis-3,5-dimethylpiperidine hydrochloride.

11. The method of Claim 1 wherein the compound is:
1-[[2,3-dihydro-6-and-7-(1,1-dimethylethyl)-1,4-benzodioxan-2-yl]methyl]-cis-3,5-dimethylpiperidine hydrochloride.

12. The method of Claim 1 wherein the compound is:
cis-1-[[6- and 7-(1,1-dimethylethyl)-2,3-dihydro-3-methyl-1,4-benzodioxan-2-yl]methyl]-cis-3,5-dimethylpiperidine.

13. The method of Claim 1 wherein the compound is:
4-[2-[6-and 7-(1,1-dimethylethyl)-2,3-dihydro-1,4-benzodioxan-2-yl]ethyl]-cis-2,6-dimethylmorpholine.

14. The compound of Formula II

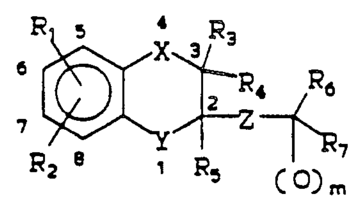

II

wherein
$R_1$ and $R_2$ are independently H; halogen; $C_1$-$C_8$ alkyl; $C_2$-$C_8$ alkenyl; $C_2$-$C_8$ alkynyl; $C_1$-$C_8$ alkoxy; $C_1$-$C_5$ alkyl substituted with 1-3 F, Cl or Br or $C_2$-$C_4$ alkoxyalkyl; $Si(CH_3)_3$; thioalkyl; or phenyl substituted with one or two of the following: halogen, $C_1$-$C_4$ alkyl or $C_1$-$C_4$ alkoxy;
$R_3$, $R_4$ and $R_5$ are independently H, $CH_3$, $CF_3$ or $CH_2CH_3$;
$R_6$ and $R_7$ are independently $C_1$-$C_8$ alkyl; or $R_6$ and $R_7$ can be taken together as piperidino, morpholino, pyrrolidino, hexamethyleneimino, heptamethyleneimino, wherein these groups are optionally substituted by 1 or 2 are of the following: $C_1$-$C_4$ alkyl, or phenyl rings substituted by hydrogen or 1 or 2 halogen, $C_1$-$C_4$ alkyl, or $C_1$ or $C_4$ alkoxy groups.
X is O or S;
Y is O or S;
Z is $C_1$-$C_4$ unbranched alkylene optionally substituted by 1-2 $CH_3$, $CF_3$ or $CH_2CH_3$ groups;

m is O or 1; and
their agriculturally suitable salts provided that

(a) when X and Y are both O, and Z is unbranched alkylene, then $R_6$ and $R_7$ are taken together as piperidino substituted at the 2, 3, 5 or 6 positions, or as substituted morpholine;

(b) when X and Y are both O, Z is $CHCH_3$, and $R_1$ and $R_2$ are both H, then $R_6$ and $R_7$ are taken together as substituted piperidino or morpholino.

15. A compound of Claim 14 wherein
$R_1$ and $R_2$ are located at the 6 and 7 positions of the benzene ring; and
$R_3$, $R_4$ and $R_5$ are independently H or $CH_3$.

16. A compound of Claim 15 wherein
$R_1$ is H, $R_2$ is $C_1$-$C_4$ alkyl, $C_1$-$C_4$ alkoxy, $C_1$-$C_4$ alkoxyalkyl or halogen; and
$R_6$ and $R_7$ are taken together as piperidino, morpholino or pyrrolidino optionally substituted by phenyl or 1-2 $C_1$-$C_2$ alkyl groups.

17. A compound of Claim 16 wherein
Z is $C_1$-$C_3$ unbranched alkylene optionally substituted by a $CH_3$ group.

18. A compound of Claim 17 wherein
$R_6$ and $R_7$ are taken together as piperidino, morpholino, pyrrolidino, 2,6-dimethylmorpholino, 3,5-dimethylpiperidino and 4-phenylpiperidino;
$R_2$ is $C_2$-$C_4$ alkyl or $C_2$-$C_4$ alkoxyalkyl;
X and Y are O.

19. The compound of Claim 1 which is:
1-[2-[6-and-7-(1,1-dimethylethyl)-2,3-dihydro-1,4-benzodioxan-2-yl]ethyl]-trans-3,5-dimethylpiperidine.

20. The compound of Claim 1 which is:
1-[2-[6-and-7-(1,1-dimethylethyl)-2,3-dihydro-1,4-benzodioxan-2-yl]ethyl]-cis-3,5-dimethylpiperidine.

21. The compound of Claim 1 which is:
1-[[2,3-dihydro-6-and-7-(1,1-dimethylethyl)-1,4-benzodioxan-2-yl]methyl]-cis-3,5-dimethylpiperidine.

22. The compound of Claim 1 which is:
1-[[6-(1,1-dimethylethyl)-2,3-dihydro-1,4-benzodioxan-2-yl]methyl]piperidine.

23. The compound of Claim 1 which is:
1-[2-[6-and-7-(1,1-dimethylethyl)-2,3-dihydro-1,4-benzodioxan-2-yl]ethyl]-cis-3,5-dimethylpiperidine hydrochloride.

24. The compound of Claim 1 which is:
1-[[2,3-dihydro-6-and-7-(1,1-dimethylethyl)-1,4-benzodioxan-2-yl]methyl]-cis-3,5-dimethylpiperidine hydrochloride.

25. The compound of Claim 1 which is:
cis-1-[[6- and 7-(1,1-dimethylethyl)-2,3-dihydro-3-methyl-1,4-benzodioxan-2-yl]methyl]-cis-3,5-dimethylpiperidine.

26. The compound of Claim 1 which is:
4-[2-[6-and 7-(1,1-dimethylethyl)-2,3-dihydro-1,4-benzodioxan-2-yl]ethyl]-cis-2,6-dimethylmorpholine.

27. A composition for controlling fungus disease which comprises an effective amount of a compound of Claim 14 and at least one of the following: surfactant, solid or liquid inert diluent.

28. A composition for controlling fungus disease which comprises an effective amount of a compound of Claim 15 and at least one of the following: surfactant, solid or liquid inert diluent.

29. A composition for controlling fungus disease which comprises an effective amount of a compound of Claim 16 and at least one of the following: surfactant, solid or liquid inert diluent.

30. A composition for controlling fungus disease which comprises an effective amount of a compound of Claim 17 and at least one of the following: surfactant, solid or liquid inert diluent.

31. A composition for controlling fungus disease which comprises an effective amount of a compound of Claim 18 and at least one of the following: surfactant, solid or liquid inert diluent.

32. A composition for controlling fungus disease which comprises an effective amount of a compound of Claim 19 and at least one of the following: surfactant, solid or liquid inert diluent.

33. A composition for controlling fungus disease which comprises an effective amount of a compound of Claim 20 and at least one of the following: surfactant, solid or liquid inert diluent.

34. A composition for controlling fungus disease which comprises an effective amount of a compound of Claim 21 and at least one of the following: surfactant, solid or liquid inert diluent.

35. A composition for controlling fungus disease which comprises an effective amount of a compound of Claim 22 and at least one of the following: surfactant, solid or liquid inert diluent.

36. A composition for controlling fungus disease which comprises an effective amount of the compound

of Claim 23 and at least one of the following: surfactant, solid or liquid inert diluent.

37. A composition for controlling fungus disease which comprises an effective amount of the compound of Claim 24 and at least one of the following: surfactant, solid or liquid inert diluent.

38. A composition for controlling fungus disease which comprises an effective amount of the compound of Claim 25 and at least one of the following: surfactant, solid or liquid inert diluent.

39. A composition for controlling fungus disease which comprises an effective amount of the compound of Claim 26 and at least one of the following: surfactant, solid or liquid inert diluent.

European Patent Office

# EUROPEAN SEARCH REPORT

Application Number

EP 89 30 8246

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | US-A-4 039 676 (CH. HUEBNER)<br>* Columns 7-16 * | 14 | C 07 D 319/20<br>A 01 N 43/32<br>C 07 D 327/06<br>C 07 D 405/06<br>C 07 D 413/06 |
| X | FR-A-1 312 892 (GEIGY)<br>* Pages 1,3; first column; claims * | 14 | |
| D,A | FR-A-2 583 266 (I.C.I.)<br>* Whole document * | 1,27-39 | |
| D,A | JOURNAL OF MEDICINAL AND PHARMACEUTICAL CHEMISTRY, vol. 5, November, 1962, pages 1285-1293; D. Misiti et al.: "Researches in the series of 1,4-benzodioxane. XXIV. Synthesis and pharmacological properties of some 2-(1-aminoethyl)-1,4-benzodioxanes"<br>* Pages 1285,1286 table I; pages 1290-1292 * | 14 | |

| TECHNICAL FIELDS SEARCHED (Int. Cl.5) |
|---|
| C 07 D 319/20<br>C 07 D 327/00<br>C 07 D 405/00<br>C 07 D 413/00 |

The present search report has been drawn up for all claims

| Place of search | Date of completion of the search | Examiner |
|---|---|---|
| THE HAGUE | 27-11-1989 | FRANCOIS J.C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document